# EUROPEAN PATENT APPLICATION

(11) **EP 4 631 926 A1**
(43) Date of publication of application: **15.10.2025**
(21) Application number: 23900464.1
(22) Date of filing: 22.11.2023
(51) Int. Cl.: C07B 37/04, C07B 61/00, C07C 1/26, C07C 15/14, C07C 15/38, C07C 15/52, C07C 41/30, C07C 43/205, C07C 45/68, C07C 49/784, C07C 67/343, C07C 69/76, C07C 209/68, C07C 211/54, C07C 253/30, C07C 255/51, C07D 209/82, C07D 295/135, C07D 333/52, C07D 333/76

(54) **METHOD FOR PRODUCING HOMO-COUPLING REACTION PRODUCT**

(30) Priority: 05.12.2022 JP 2022194076
(71) Applicant: National University Corporation Hokkaido University, Hokkaido 060-0808 (JP)
(72) Inventor: ITO, Hajime, Sapporo-shi, Hokkaido 060-0808 (JP); KUBOTA, Koji, Sapporo-shi, Hokkaido 060-0808 (JP)
(74) Representative: Hasegawa, Kan
(86) International application number: PCT/JP2023/042007
(87) International publication number: WO 2024/122345

(57) **Abstract**

An object is to provide a method for producing a homocoupling reaction product, which can be carried out substantially without the use of organic solvents, allows a reaction to be carried out under mild reaction conditions by a simple reaction operation, allows a wide range of organic halogen compounds to be used as raw materials, can provide a reaction product in a short time in high yield, and can provide a reaction product having a high molecular weight. Provided as a solution is a method for producing a homocoupling reaction product of an organic halogen compound represented by formula (I), the method including subjecting the organic halogen compound represented by formula (I);

A¹-Xₘ (I)

(In formula (I), A¹ represents an optionally substituted **m-**valent aromatic hydrocarbon group, an optionally substituted m-valent aromatic heterocyclic group, an optionally substituted m-valent heterocyclic group, an optionally substituted m-valent aliphatic hydrocarbon group, or an optionally substituted m-valent unsaturated aliphatic hydrocarbon group. X represents chlorine, bromine, or iodine, and when a plurality of **X's** are present, they may be identical to or different from each other. m is the number of X and represents an integer of 1 or more.)
to a reaction in the presence of a nickel catalyst under a condition (a) and/or a condition (b);
Condition (a): a condition where the amount of a solvent used is 0.8 mL or less relative to 1 mmol of the organic halogen compound,
Condition (b): a condition where a heteroatom-containing compound is present,
by a mechanochemical method.

## Description

### Technical Field

The present invention relates to a novel method for producing a homocoupling reaction product.

### Background Art

As means for synthesizing functional materials such as pharmaceuticals, liquid crystal compounds, organic electroluminescence compounds, organic thin-film solar cells, macromolecular compounds, oligomers, coloring materials, radiation absorbing materials, information recording materials, wavelength conversion materials, indicator materials, sensor materials, organic light-emitting diodes (OLEDs), and organic semiconductor materials, homocoupling reactions are known. Homocoupling reactions, which are reactions in which molecules of the same type are bonded together, are used to synthesize various compounds. In particular, a homocoupling reaction of a halogen compound in the presence a nickel catalyst (Yamamoto homocoupling reaction) is used for, for example, the synthesis of a compound by carbon-carbon bond formation reaction and the polymerization reaction of a π-conjugated polymer.

NPL 1 discloses a reaction in which aromatic halogen compounds are homocoupled using a zero-valent nickel complex, thereby undergoing a dehalogenation reaction to produce a biaryl compound. NPLs 2 and 3 disclose that polypyridines and the like can be synthesized in high yield by performing dehalogenation polycondensation via homocoupling reaction using a zero-valent nickel catalyst using an aromatic dihalogen compound and that the dehalogenation polycondensation is applicable to a very wide range of monomers.
PTL 1 discloses a method for producing a cyclic aromatic compound. In the method, a zero-valent nickel catalyst and a m-dihalogenated benzene such as 1,3-dibromobenzene are mixed together to polymerize the m-dihalogenated benzene via the Yamamoto coupling reaction, thus obtaining a cyclic aromatic compound.
PTL 2 discloses a method for producing a copolymer by polymerizing a dihalogen compound monomer (A) and a dihalogen compound macromer (B) in the coexistence of zinc (C) and a divalent nickel complex (D) catalyst.
PTL 3 discloses a cross-coupling reaction method in which an organic compound (A) that has a melting point of 30°C or higher and has a leaving group and an organic compound (B) that reacts with an organic compound having a leaving group and has a melting point of 30°C or higher are reacted with each other in the presence of a catalyst by a mechanochemical method in which mechanical energy is applied to the organic compound (A) and the organic compound (B) under the following conditions: the amount of organic solvent, 0.7 mL or less per mmol of the total amount of the organic compound (A) and the organic compound (B); temperature, 60°C to 500°C.

### Citation List

### Non Patent Literature

NPL 1: M. F. Semmelhack, et al, J. Am. Chem. Soc., 1971, Vol. 93, No. 22, 5908-5910
NPL 2: T, Yamamoto, et al, Chem. Lett., 1988, Vol. 17, No. 1, 153-154,
NPL 3: T. Yamamoto, et al, Macromolecures, 1992, Vol. 25, No. 4, 1214-1223

### Patent Literature

PTL 1: Japanese Unexamined Patent Application Publication No. 2016-32083
PTL 2: Japanese Unexamined Patent Application Publication No. 2019-19226
PTL 3: International Publication No. 2022/092260

### Summary of Invention

### Technical Problem

Homocoupling reactions generally require relatively large amounts of organic solvents because the reactions are carried out with starting compounds dissolved in organic solvents. However, in recent years, the use of large amounts of organic solvents may cause problems in terms of the work environment and safety of workers, global environmental protection, environmental burden during the treatment of used organic solvents, etc. In addition, for reasons of, for example, the stability of nickel catalysts for use, stringent reaction conditions are required, it takes long reaction times, and reaction operations are complicated. Furthermore, yields have been unsatisfactory.

An object of the present invention is to provide a method for producing a homocoupling reaction product, which can be carried out substantially without the use of organic solvents, allows a reaction to be carried out under mild reaction conditions by a simple reaction operation, allows a wide range of organic halogen compounds to be used as raw materials, can provide a reaction product in a short time in high yield, and can provide a reaction product having a high molecular weight.

### Solution to Problem

The present inventors have conducted intensive studies and found that the above object can be achieved by carrying out a homocoupling reaction under specific conditions by a mechanochemical method, thereby completing the present invention.

Thus, the present invention provides the following method for producing a homocoupling reaction product.
[Item 1]A method for producing a homocoupling reaction product of an organic halogen compound represented by formula (I), the method including subjecting the organic halogen compound represented by formula (I);

   A¹-Xₘ (I)

   (In formula (I),
   A¹ represents an optionally substituted m-valent aromatic hydrocarbon group, an optionally substituted m-valent aromatic heterocyclic group, an optionally substituted m-valent heterocyclic group, an optionally substituted m-valent aliphatic hydrocarbon group, or an optionally substituted m-valent unsaturated aliphatic hydrocarbon group.
   X represents chlorine, bromine, or iodine, and when a plurality of X's are present, they may be identical to or different from each other.
   m is a number of X and represents an integer of 1 or more.) to a reaction in presence of a nickel catalyst under a condition (a) and/or a condition (b);
   Condition (a): a condition where an amount of a solvent used is 0.8 mL or less relative to 1 mmol of the organic halogen compound,
   Condition (b): a condition where a heteroatom-containing compound is present,
   by a mechanochemical method.
[Item 2]The method for producing a homocoupling reaction product according to Item 1, wherein
   in formula (I), m is 1 or more and 4 or less, and
   the homocoupling reaction product is represented by formula (A), formula (P1), formula (P2), or formula (P3);
   a homocoupling reaction product represented by formula (A);

      A¹-A¹ (A)

      (In formula (A), A¹ may be identical or different.),
         a linear homocoupling reaction polymerization product, cyclic homocoupling reaction polymerization product, or intramolecular homocoupling reaction product having a repeating unit represented by
         formula (P1); 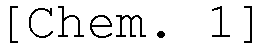 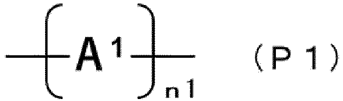
      (In formula (P1), a plurality of A¹'s may be identical or different, and n1 is an integer of 2 or more.),
         a polymer having a unit represented by
         formula (P2); 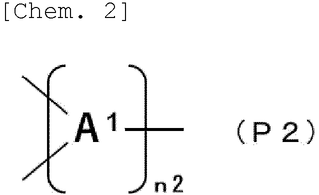
      (In formula (P2), a plurality of A¹'s may be identical or different, and n2 is an integer of 2 or more.),
         a polymer having a unit represented by
         formula (P3); 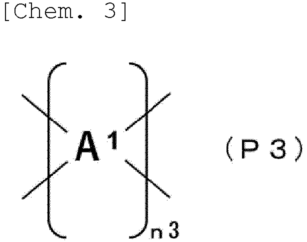
      (In formula (P3), a plurality of A¹'s may be identical or different, and n3 is an integer of 2 or more.).
[Item 3]The method for producing a homocoupling reaction product according to Item 1 or 2, wherein the solvent has a melting point of 30°C or lower, and/or the heteroatom-containing compound has a melting point of 50°C or higher.
[Item 4]The method for producing a homocoupling reaction product according to any one of Items 1 to 3, wherein a reductant is further present in the reaction by the mechanochemical method.
[Item 5]The method for producing a homocoupling reaction product according to any one of Items 1 to 4, wherein a temperature in the reaction by the mechanochemical method is 60°C or higher.

### Advantageous Effects of Invention

The present invention provides a method for producing a homocoupling reaction product, which can be carried out substantially without the use of organic solvents, allows a reaction to be carried out under mild reaction conditions by a simple reaction operation, allows a wide range of organic halogen compounds to be used as raw materials, can provide a reaction product in a short time in high yield, and can provide a reaction product having a high molecular weight. Description of Embodiments

### [Method for producing homocoupling reaction product]

A method for producing a homocoupling reaction product according to the present invention is a method for producing a homocoupling reaction product, the method including subjecting an organic halogen compound represented by formula (I);

A¹-Xₘ (I)

(In formula (I),
A¹ represents an optionally substituted m-valent aromatic hydrocarbon group, an optionally substituted m-valent aromatic heterocyclic group, an optionally substituted m-valent heterocyclic group, an optionally substituted m-valent aliphatic hydrocarbon group, or an optionally substituted m-valent unsaturated aliphatic hydrocarbon group.
X represents chlorine, bromine, or iodine, and when a plurality of X's are present, they may be identical to or different from each other.
m is the number of X and represents an integer of 1 or more.)
to a reaction in the presence of a nickel catalyst under a condition (a) and/or a condition (b);
Condition (a): a condition where the amount of a solvent used is 0.8 mL or less relative to 1 mmol of the organic halogen compound,
Condition (b): a condition where a heteroatom-containing compound is present, by a mechanochemical method.

The method for producing a homocoupling reaction product according to the present invention may be a method for producing a homocoupling reaction product, in which the reaction is carried out by the mechanochemical method under the condition (a) alone.

The method for producing a homocoupling reaction product according to the present invention may be a method for producing a homocoupling reaction product, in which the reaction is carried out by the mechanochemical method under the condition (b) alone.

The method for producing a homocoupling reaction product according to the present invention may be a method for producing a homocoupling reaction product, in which the reaction is carried out by the mechanochemical method under both the condition (a) and the condition (b).

### <Organic halogen compound>

In the method for producing a homocoupling reaction product according to the present invention, the organic halogen compound may be a compound represented by formula (I);

A¹-Xₘ (I)

**(**In formula (I),
A¹ represents an optionally substituted m-valent aromatic hydrocarbon group, an optionally substituted m-valent aromatic heterocyclic group, an optionally substituted m-valent heterocyclic group, an optionally substituted m-valent aliphatic hydrocarbon group, or an optionally substituted m-valent unsaturated aliphatic hydrocarbon group.
X represents chlorine, bromine, or iodine, and when a plurality of X's are present, they may be identical to or different from each other.
m is the number of X and represents an integer of 1 or more.).

As the organic halogen compound represented by formula (I), one compound can be used alone, or two or more compounds can be used in combination. As the organic halogen compound represented by formula (I), a commercially available product can be used directly or after being purified.

### (A¹ group in formula (I))

The number of carbon atoms in the optionally substituted m-valent aromatic hydrocarbon group as the A¹ group is not particularly limited, and is, for example, 6 to 60, preferably 6 to 40, more preferably 6 to 30.

In the m-valent aromatic hydrocarbon group, m is an integer of 1 or more, for example, 1 to 10, preferably 1 to 6, more preferably 1 to 4.

For the optionally substituted m-valent aromatic hydrocarbon group as the A¹ group, examples of monovalent aromatic hydrocarbon groups in the case where m = 1 include a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group), a triphenylenyl group (or a triphenylene group), a fluorenyl group, and a spirobifluorenyl group.

For the optionally substituted m-valent aromatic hydrocarbon group as the A¹ group, examples of m-valent aromatic hydrocarbon groups in the case where m is an integer of 2 or more include groups obtained by removing (m - 1) hydrogen atoms from aromatic rings in the above monovalent aromatic hydrocarbon groups.

The number of carbon atoms in the optionally substituted m-valent aromatic heterocyclic group as the A¹ group is not particularly limited, and is, for example, 4 to 60, preferably 4 to 40, more preferably 4 to 30. In the m-valent aromatic heterocyclic group as the A¹ group, m is an integer of 1 or more, for example, 1 to 10, preferably 1 to 6, more preferably 1 to 4. For the optionally substituted m-valent aromatic heterocyclic group as the A¹ group, examples of monovalent aromatic heterocyclic groups in the case where m = 1 include sulfur-containing heteroaryl groups such as a thiophenyl group (a thiophene group or a thienyl group), a benzothienyl group (a benzothiophene group), and a dibenzothienyl group (a dibenzothiophene group); oxygen-containing heteroaryl groups such as a furanyl group (or a furan group), a benzofuranyl group (a benzofuran group), a dibenzofuranyl group (a dibenzofuran group), a phenyldibenzofuranyl group, and a dibenzofuranylphenyl group; nitrogen-containing heteroaryl groups such as a pyridyl group (or a pyridine group), a pyrimidinyl group (or a pyrimidine group), a pyrazyl group (or a pyrazine group), a quinolyl group (or a quinoline group), an isoquinolyl group (or an isoquinoline group), a carbazolyl group (or a carbazole group), a 9-phenylcarbazolyl group, an acridinyl group (or an acridine group), a quinazolyl group (or a quinazoline group), a quinoxalyl group (or a quinoxaline group), a 1,6-naphthyridinyl group, a 1,8-naphthyridinyl group, and a porphyrin group (or a porphyrin ring); and heteroaryl groups containing two or more heteroatoms (e.g., nitrogen and sulfur), such as a benzothiazolyl group (or a benzothiazole group) and a benzothiadiazole group. Further examples include a pyrrole group, a silole group, a borole group, a phosphole group, a selenophene group, a germole group, an indole group, an indene group, a benzosilole group, a benzoborole group, a benzophosphole group, a benzoselenophene group, a benzogermole group, a dibenzosilole group, a dibenzoborole group, a dibenzophosphole group, a dibenzoselenophene group, a dibenzogermole group, a dibenzothiophene 5-oxide group, a 9H-fluoren-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluoren-9-one group, an azadibenzothiophene 5,5-dioxide group, a pyridazine group, a triazine group, a phenanthroline group, a pyrazole group, an imidazole group, a triazole group, an oxazole group, an isoxazole group, a thiazole group, an isothiazole group, an oxadiazole group, a thiadiazole group, a benzopyrazol group, a benzoimidazole group, a benzoxazole group, a benzooxadiazole group, a 5,6,7,8-tetrahydroisoquinoline group, and a 5,6,7,8-tetrahydroquinoline group.

For the optionally substituted m-valent aromatic heterocyclic group as the A¹ group, examples of m-valent aromatic heterocyclic groups in the case where m is an integer of 2 or more include groups obtained by removing (m - 1) hydrogen atoms from aromatic rings in the above monovalent aromatic heterocyclic groups. Other examples include a benzo[1,2-c:4,5-c']bis[1,2,5]thiadiazole skeleton (a benzobisthiadiazole group), a thienylenyl group (or a thiophenediyl group), a phenyldibenzothienylenyl group, a dibenzothienylenylphenyl group, and a pyridylenyl group (or a pyridinediyl group).

The number of carbon atoms in the optionally substituted m-valent aliphatic hydrocarbon group as the A¹ group is not particularly limited, and is, for example, 2 to 60, preferably 3 to 40, more preferably 5 to 30.

In the m-valent aliphatic hydrocarbon group as the A¹ group, m is an integer of 1 or more, for example, 1 to 10, preferably 1 to 6, more preferably 1 to 4.

For the optionally substituted m-valent aliphatic hydrocarbon group as the A¹ group, examples of monovalent aliphatic hydrocarbon groups in the case where m = 1 include saturated aliphatic hydrocarbon groups such as alkyl groups and cycloolefin groups.

For the optionally substituted m-valent aliphatic hydrocarbon group as the A¹ group, examples of m-valent aliphatic hydrocarbon groups in the case where m is an integer of 2 or more include groups obtained by removing (m - 1) hydrogen atoms from the above monovalent aliphatic hydrocarbon groups.

In the main chain or substituent of the optionally substituted m-valent aliphatic hydrocarbon group as the A¹ group, a heteroatom (e.g., nitrogen, oxygen, phosphorus, or sulfur) may be contained. Examples of heteroatom-containing substituents include the above-mentioned aromatic heterocyclic groups such as a thiophenyl group, a furanyl group, and a pyrrole group, and saturated heterocyclic groups such as a tetrahydrothienyl group, a tetrahydrofuranyl group, a pyrrolidinyl group, a piperazyl group, and a morpholyl group.

The number of carbon atoms in the optionally substituted m-valent unsaturated aliphatic hydrocarbon group as the A¹ group is not particularly limited, and is, for example, 2 to 60, preferably 3 to 40, more preferably 5 to 30.

In the m-valent unsaturated aliphatic hydrocarbon group as the A¹ group, m is an integer of 1 or more, for example, 1 to 10, preferably 1 to 6, more preferably 1 to 4.

For the optionally substituted m-valent unsaturated aliphatic hydrocarbon group as the A¹ group, examples of monovalent aromatic hydrocarbon groups in the case where m = 1 include alkenyl groups and alkynyl groups.

For the optionally substituted m-valent unsaturated aliphatic hydrocarbon group as the A¹ group, examples of m-valent unsaturated aliphatic hydrocarbon groups in the case where m is an integer of 2 or more include groups obtained by removing (m - 1) hydrogen atoms from the above monovalent unsaturated aliphatic hydrocarbon groups.

In the main chain or substituent of the optionally substituted m-valent unsaturated aliphatic hydrocarbon group as the A¹ group, a heteroatom (e.g., nitrogen, oxygen, phosphorus, or sulfur) may be contained. Examples of heteroatom-containing substituents include the above-mentioned aromatic heterocyclic groups such as a thiophenyl group, a furanyl group, and a pyrrole group, and saturated heterocyclic groups such as a tetrahydrothienyl group, a tetrahydrofuranyl group, a pyrrolidinyl group, a piperazyl group, and a morpholyl group.

In the organic halogen compound represented by formula (I), examples of A¹ in formula (I) include the following groups.

Phenyl groups (e.g., alkyl (e.g., methyl) phenyl groups, dialkyl (e.g., dimethyl) phenyl groups, alkoxy (e.g., methoxy) phenyl groups, dialkylamino (e.g., dimethylamino) phenyl groups, diaryl (e.g., diphenyl) aminophenyl groups, perfluoroalkyl (e.g., trifluoromethyl) phenyl groups, alkyl (e.g., ethyl) oxycarbonylphenyl groups, and alkanoyl (e.g., acyl) phenyl groups), and phenyl groups bridged by a linking group such as an alkylene group, an ether group, or an ester group;
naphthyl groups such as a naphthyl group, aryl (e.g., phenyl) naphthyl groups, alkylene-bridged (e.g., ethylene-bridged) naphthyl groups, and arylene-bridged (e.g., phenylene-bridged) naphthyl groups;
a phenanthrenyl group;
anthracenyl groups such as an anthracenyl group, aryl (e.g., phenyl) anthracenyl groups, diaryl (e.g., dinaphthyl) anthracenyl groups, and diarylboryl (e.g., bis(trialkylphenyl)boryl) anthracenyl groups;
pyrenyl groups such as a pyrenyl group and alkyl (e.g., tert-butyl) pyrenyl groups;
biphenyl groups such as a biphenyl group and alkylene-bridged (e.g., propylene-bridged, isopropylene-bridged) biphenyl groups;
terphenyl groups such as a terphenyl group and tetraaryl (e.g., tetraphenyl) terphenyl groups;
a triphenylenyl group;
a fluorenyl group and a spirobifluorenyl group;
2-aryl (e.g., phenyl) ethenylphenyl groups, 1,2,2-triaryl (e.g., triphenyl) ethenylphenyl groups, and 2-aryl (e.g., phenyl) ethenylphenyl groups;
aryl-substituted (e.g., phenyl-substituted) carbazolyl groups;
an anthracene-9.10-dione group;
aryl-substituted (e.g., phenyl-substituted) thienyl groups, a thiophene group, and a benzothiadiazole group; and
groups having a valence of 2 or more, such as a phenylene group, aryl (e.g., bis(3,5-methylphenyl)) porphyrin rings, a pyren-tetra-yl group, and a benzo[1,2-c:4,5-c']bis[1,2,5]thiadiazole skeleton (a benzobisthiadiazole group).

The optional substituent in the optionally substituted m-valent aromatic hydrocarbon group, the optionally substituted m-valent aromatic heterocyclic group, the optionally substituted m-valent aliphatic hydrocarbon group, or the optionally substituted m-valent unsaturated aliphatic hydrocarbon group as the A¹ group is not particularly limited as long as the homocoupling reaction aimed at by the present invention can be carried out.

Examples of the substituent include one or more selected from the group consisting of alkyl groups having 1 to 24, preferably 1 to 18, more preferably 1 to 12, still more preferably 1 to 8 carbon atoms (e.g., a methyl group, an ethyl group, a n-propyl group, an isopropyl group, a n-butyl group, an isobutyl group, a tert-butyl group, a pentyl group, a hexyl group, and an octyl group); alkoxy groups having 1 to 24, preferably 1 to 18, more preferably 1 to 12, still more preferably 1 to 8 carbon atoms (e.g., a methoxy group, an ethoxy group, a n-propoxy group, an isopropoxy group, a n-butoxy group, an isobutoxy group, a tert-butoxy group, a pentyloxy group, a hexyloxy group, and an octyloxy group); cycloalkyl groups having 3 to 24, preferably 3 to 18, more preferably 3 to 12, still more preferably 3 to 8 carbon atoms (e.g., a cyclopropyloxy group, a cyclobutyloxy group, a cyclopentyloxy group, and a cyclohexyloxy group); alkenyl groups having 1 to 24, preferably 1 to 18, more preferably 1 to 12, still more preferably 1 to 8 carbon atoms (e.g., an ethenyl group, a propenyl group, a butenyl group, a pentenyl group, a hexenyl group, and an octenyl group); alkynyl groups having 1 to 24, preferably 1 to 18, more preferably 1 to 12, still more preferably 1 to 8 carbon atoms (e.g., an ethynyl group, a propynyl group, a butynyl group, a pentynyl group, a hexynyl group, and an octynyl group); aryl groups having 5 to 24, preferably 5 to 18, more preferably 5 to 12, still more preferably 5 to 8 carbon atoms (e.g., a phenyl group, a naphthyl group, and a biphenyl group); arylalkyl groups having 7 to 24, preferably 7 to 19, more preferably 7 to 13, still more preferably 7 to 9 carbon atoms (e.g., a monophenylmethyl group, a monophenylpropyl group, and a triphenylmethyl group); aryloxy groups having 5 to 24, preferably 5 to 18, more preferably 5 to 12, still more preferably 5 to 8 carbon atoms (e.g., a phenoxy group, a naphthyloxy group, and a biphenyloxy group); heteroaryl groups having 4 to 24, preferably 4 to 18, more preferably 4 to 12, still more preferably 4 to 8 carbon atoms (e.g., a thiophenyl group, a furanyl group, a carbazole group, a benzothiophenyl group, a benzofuranyl group, an indolyl group, a pyrrolyl group, and a pyridyl group); acyl groups having 1 to 24, preferably 1 to 18, more preferably 1 to 12, still more preferably 1 to 8 carbon atoms (e.g., an acetyl group, a propionyl group, a butanoyl group, a pentanoyl group, a heptanoyl group, and groups obtained by substituting carbonyl groups contained in these acyl groups with ester groups or amide groups); amino groups having 1 to 24, preferably 1 to 18, more preferably 1 to 12, still more preferably 1 to 8 carbon atoms (e.g., a diphenylamino group and a dimethylamino group); fluorine and fluorine-containing groups such as fluorine-containing hydrocarbon groups having 1 to 30, preferably 1 to 12 carbon atoms; a cyano group and a nitro group; and the like.

The substituents may be mutually bridged, and the whole substituents may form a cyclic structure (an aromatic group). The substituents may further have a substituent.

### (X group in formula (I))

The X group represents chlorine, bromine, or iodine, and when a plurality of X's are present, they may be identical to or different from each other. An appropriate group can be used depending on reactivity, etc.

The number m of X groups in formula (I) is an integer of 1 or more, and is not particularly limited as long as the homocoupling reaction can be carried out. For example, m can be 10 or less, preferably 8 or less, more preferably 6 or less, still more preferably 4 or less.

### <Nickel catalyst>

The nickel complex used in the method for producing a homocoupling reaction product according to the present invention is not particularly limited as long as it can catalyze (accelerate) the homocoupling reaction of the halogen compound. The nickel catalyst is preferably a zero-valent nickel catalyst including zero-valent nickel or a divalent nickel catalyst including divalent nickel. The nickel catalyst is more preferably a catalyst that can form zero-valent nickel (Ni(0)) in the reaction system. Examples of the nickel catalyst include one or more selected from the group consisting of zero-valent nickel complexes including Ni(0), divalent nickel complexes including Ni(II), salts of zero-valent Ni, salts of divalent Ni, etc.

The nickel catalyst may be a mixture of one or more zero-valent nickel catalysts and one or more divalent nickel catalysts. When a divalent nickel complex is used, it can be used in combination with a reductant described later.

The nickel catalyst can be used alone or in combination of two or more. As the nickel catalyst, for reasons of availability, a commercially available product can be used directly or after being purified. Furthermore, the nickel catalyst can be used, if necessary, in a state of being supported on a carrier such as alumina, carbon, silica, or zeolite.

Examples of zero-valent nickel catalysts include one or more selected from the group consisting of bis(1,5-cyclooctadiene) nickel (0) [Ni (cod)₂] tetrakis(triphenylphosphine)nickel(0), tetracarbonylnickel(0), dicarbonylbis(triphenylphosphine)nickel(0), and hydrates thereof.

Examples of divalent nickel catalysts include one or more selected from the group consisting of dichloro [bis (triphenylphosphine)] nickel (II) [Ni(PPh₃)₂Cl₂], dibromo[bis(triphenylphosphine)] nickel (II) [Ni(PPh₃)₂Br₂], dichloro[bis(diphenylphosphino)methane]nickel(II) [Ni(dppm)Cl₂], dibromo[bis(diphenylphosphino)methane]nickel(II) [Ni(dppm)Br_{2]}, dichloro[1,2-bis(diphenylphosphino)ethane]nickel(II) [Ni(dppe)Cl₂], dibromo[1,2-bis(diphenylphosphino)ethane]nickel(II) [Ni(dppe)Br₂], dichloro[1,3-bis(diphenylphosphino)propane]nickel(II) [Ni(dppp)Cl₂], dibromo[1,3-bis(diphenylphosphino)propane]nickel(II) [Ni(dppp)Br₂], dichloro[1,1'-bis(diphenylphosphino)iron]nickel(II) [Ni(dppf)Cl₂], dibromo[1,1'-bis(diphenylphosphino)iron]nickel(II) [Ni(dppf)Br₂], dichloro[1,4-bis(diphenylphosphino)butane]nickel(II), dibromo[1,4-bis(diphenylphosphino)butane]nickel(II), dichlorobis(tributylphosphine)nickel(II), dibromobis(tributylphosphine)nickel(II), dichlorobis(trimethylphosphine)nickel(II), dibromobis(trimethylphosphine)nickel(II), dichloro(ethylenediamine)nickel, dibromo(ethylenediamine)nickel, dichloro(N,N,N',N'-tetramethylethylenediamine)nickel(II), dibromo(N,N,N',N'-tetramethylethylenediamine)nickel(II), dichloro[bis(tri-n-butylphosphine)]nickel(II), dibromo[bis(tri-n-butylphosphine)]nickel(II), dichloro[bis(cyclohexyldiphenylphosphine)]nickel(II), dibromo[bis(cyclohexyldiphenylphosphine)]nickel(II), dichloro[bis(tricyclohexylphosphine)]nickel(II), dibromo[bis(tricyclohexylphosphine)]nickel(II), dichloro[bis(trianisoylphosphine)]nickel(II), dibromo[bis(trianisoylphosphine)]nickel(II), dichloro[2,3-bis(2,6-diisopropylphenylimino)butane]nickel(II), dibromo[2,3-bis(2,6-diisopropylphenylimino)butane]nickel(II), chloro(2-methylphenyl)bis(triphenylphosphine)nickel(II), bromo(2-methylphenyl)bis(triphenylphosphine)nickel(II), chloro[bis[(2-dimethylamino)phenyl]amine]nickel(II), bromo[bis[(2-dimethylamino)phenyl]amine]nickel(II), chloro(cyclopentadienyl)(triphenylphosphine)nickel(II), bromo(cyclopentadienyl)(triphenylphosphine)nickel(II), chloro (ethylcyclopentadienyl) (triphenylphosphine) nickel (II), bromo(ethylcyclopentadienyl)(triphenylphosphine)nickel(II), chloro(2-methylphenyl)bis(triphenylphosphine)nickel(II), bromo(2-methylphenyl)bis(triphenylphosphine)nickel(II), chloro(1-naphthyl)bis(triphenylphosphine)nickel(II), bromo(1-naphthyl)bis(triphenylphosphine)nickel(II), bis(isopropylcyclopentadienyl)nickel, bis(methylcyclopentadienyl)nickel(II), nickel(II) acetylacetonate, methallyl nickel chloride dimer, hexaaminenickel(II) bromide, bis(trifluoromethanesulfonimide)nickel(II), nickel(II) hexafluoroacetylacetonate, bis(4-diethylaminodithiobenzyl)nickel(II), N,N'-bis(salicylidene)ethylenediaminonickel(II), bis(cyclopentadienyl)nickel(II), nickel(II) chloride, nickel(II) bromide, nickel(II) iodide, nickel(II) fluoride, nickel(II) nitrate, nickel(II) sulfate, nickel(II) carbonate, nickel(II) boride, nickel(II) borate, nickel(II) hypophosphite, nickel(II) ammonium sulfate, nickel(II) hydroxide, nickel(II) formate, nickel(II) acetate, nickel(II) trifluoroacetate, nickel(II) citrate, nickel(II) oxalate, nickel(II) cyclohexanebutyrate, nickel(II) benzoate, nickel(II) stearate, nickel(II) stearate, nickel(II) sulfamate, nickel(II) thiocyanate, nickel(II) trifluoromethanesulfonate, hydrates thereof, and the like.

In the method for producing a homocoupling reaction product according to the present invention, the amount of the nickel catalyst used is not particularly limited. Relative to 1 mol of the organic halogen compound, the amount of the nickel catalyst used is, for example, 0.01 mol or more, preferably 0.05 mol or more, more preferably 0.1 mol or more, still more preferably 0.5 mol or more, and is, for example, 50 mol or less, preferably 30 mol or less, more preferably 20 mol or less, still more preferably 10 mol or less. When the amount of the nickel catalyst used is, for example, less than 0.01 mol or more than 50 mol relative to 1 mol of the organic halogen compound, the reaction may not proceed smoothly.

### <Solvent>

The condition (a) in the method for producing a homocoupling reaction product according to the present invention is a condition where the amount of a solvent used is 0.8 mL or less relative to 1 mmol of the organic halogen compound. This condition can be said to be a substantially solvent-free condition. In the present invention, the term "substantially solvent-free condition" refers to any of a state where no solvents are used, a state where solvents are not actively used, and a state where solvents are used but in such a very small amount that the solvent effect is not exhibited.

In the method for producing a homocoupling reaction product according to the present invention, the solvent in the condition (a) is not particularly limited. The solvent is liquid at normal temperature (25°C ± 5°C) and does not react with the organic halogen compound represented by formula (I) serving as a substrate. Examples include solvents used in homocoupling reactions carried out in solution systems. Examples include one or more selected from the group consisting of oxygen-containing organic solvents such as methanol, ethanol, n-propanol, isopropanol, 1-butanol, 1,1-dimethylethanol, tert-butanol, 2-methoxyethanol, ethylene glycol, polyethylene glycol, polypropylene glycol, diethyl ether, diisopropyl ether, dibutyl ether, t-butylmethyl ether, tetrahydrofuran, tetrahydropyran, cyclopentylmethyl ether, dimethoxyethane, 1,4-dioxane, anisole, acetoxy-2-ethoxyethane, propylene glycol monomethyl ether acetate, ethylene glycol dimethyl ether, diethylene glycol dimethyl ether, polyethylene glycol dimethyl ether, polyethylene glycol diethyl ether, 1-methoxy-1,1,2,2-tetrafluoroethane, 1-ethoxy-1,1,2,2-tetrafluoroethane, acetone, methyl ethyl ketone, ethyl acetate, butyl acetate, and acetic acid; aromatic solvents such as benzene, toluene, xylene, mesitylene durene, and decalin; aliphatic organic solvents such as hexane, pentane, and heptane; halogenated hydrocarbon organic solvents such as dichloromethane, chloroform, carbon tetrachloride, chlorobenzene, and 1,2-dichlorobenzene; nitrogen-containing organic solvents such as acetonitrile, N,N'-dimethylformamide, N,N'-dimethylacetamide, N-methyl-2-pyrolidone, and pyridine; sulfur-containing organic solvents such as dimethylsulfoxide; water; and the like. The solvent is preferably a solvent having a melting point of 30°C or lower, particularly, an organic solvent having a melting point of 30°C or lower.

In the method for producing a homocoupling reaction product according to the present invention, the solvent can be used as a liquid grinding aid (Liquid Assisted Grinding: LAG) in carrying out the reaction by a mechanochemical method. Among the above solvents, the liquid grinding aid is preferably, for example, one or more selected from the group consisting of oxygen-containing organic solvents such as 2-methoxyethanol, ethylene glycol, diethyl ether, dibutyl ether, t-butylmethyl ether, tetrahydrofuran, 1,4-dioxane, propylene glycol monomethyl ether acetate, ethylene glycol dimethyl ether, diethylene glycol dimethyl ether, diethyl ether, dibutyl ether, tetrahydrofuran, ethylene glycol, polypropylene glycol, polyethylene glycol dimethyl ether, and polyethylene glycol diethyl ether; nitrogen-containing organic solvents such as N,N'-dimethylformamide, N,N'-dimethylacetamide, N-methyl-2-pyrolidone, and pyridine; sulfur-containing organic solvents such as dimethylsulfoxide; and the like. Particularly preferably, one or more selected from the group consisting of 2-methoxyethanol, ethylene glycol, diethyl ether, dibutyl ether, t-butylmethyl ether, tetrahydrofuran, N',N'-dimethylformamide, N,N'-dimethylacetamide, N-methyl-2-pyrolidone, pyridine, and dimethylsulfoxide can be used.

In the method for producing a homocoupling reaction product according to the present invention, the amount of the solvent used is 0.8 mL or less relative to 1 mmol of the organic halogen compound, preferably 0.5 mL or less, more preferably 0.3 mL or less. In the method for producing a homocoupling reaction product according to the present invention, the amount of the solvent used can be 0 mL (solvent-free).

In a typical homocoupling reaction carried out in a solution system, an organic solvent in an amount of 1 mL or more relative to 1 mmol of the total amount of reaction raw materials is used. In the present invention, since the amount of the solvent, particularly, the organic solvent used is 0.8 mL or less relative to 1 mmol of the organic halogen compound, components such as the organic halogen compound and the nickel catalyst, at the start of the reaction, are present typically in a state where at least portions thereof are not dissolved in the solvent and others, occasionally in a solid state where the whole components are not dissolved in the solvent and others, and will undergo the reaction.

### <Heteroatom-containing compound>

The condition (b) in the method for producing a homocoupling reaction product according to the present invention is a condition where a heteroatom-containing compound is present. The heteroatom means an atom other than carbon and hydrogen. In the present invention, the heteroatom is preferably a nitrogen atom, a phosphorus atom, a boron atom, or an oxygen atom. In the method for producing a homocoupling reaction product according to the present invention, the heteroatom-containing compound in the condition (b) is not particularly limited, and is preferably a heteroatom-containing compound having a melting point of 50°C or higher. Examples of the heteroatom-containing compound include one or more selected from the group consisting of bipyridine compounds such as bipyridine, 4,4'-bis(di-tert-butyl)-2,2'-bipyridine, and phenanthroline; arylphosphines such as triphenylphosphine, tri(o-tolyl)phosphine, tri(2-furyl)phosphine, tert-butyldiphenylphosphine, tris(2,5-xylyl)phosphine, and tri(mesityl)phosphine; alkylphosphine compounds such as trimethylphosphine, tributylphosphine, tri(isopropyl)phosphine, tri(tert-butyl)phosphine, and tri(cyclohexyl)phosphine; Buchwald phosphine ligand compounds such as 2-dicyclohexylphosphino-2',6'-dimethoxybiphenyl (SPhos), 2-(dicyclohexylphosphino)-2'-(N,N-dimethylamino)biphenyl (DavePhos), 2-(di-tert-butylphosphino)-2',4',6'-triisopropyl-3,6-dimethoxy-1,1'-biphenyl (tBuBrettPhos), 2-dicyclohexylphosphino-2',6'-diisopropoxybiphenyl, 2-dicyclohexylphosphino-2'-methylbiphenyl, 2-dicyclohexylphosphino-2',4',6'-triisopropylbiphenyl, 2-(di-tert-butylphosphino)-2',4',6'-triisopropylbiphenyl, 2-dicyclohexylphosphino-3,6-dimethoxy-2',4',6'-triisopropylbiphenyl, 2-(dicyclohexylphosphino)biphenyl, and 2-(di-tert-butylphosphino)-2'-(N,N-dimethylamino)biphenyl; bidentate phosphine compounds such as 2-(di-tert-butylphosphino)biphenyl, 2-(dicyclohexylphosphino)biphenyl, 9,9-dimethyl-4,5-bis(diphenylphosphino)xanthene, bis(diphenylphosphino)methane, 1,2-bis(diphenylphosphino)ethane, 1,2-bis(diphenylphosphino)propane, 1,2-bis(dicyclohexylphosphino)ethane, 1,2-bis(diphenylphosphino)butane, 1,4-bis(diphenylphosphino)butane, 2,2-dimethyl-1,3-bis(diphenylphosphino)propane, 1,1'-bis(diphenylphosphino)ferrocene, and 1,2-bis(diphenylphosphino)ferrocene; N-heterocarbene compounds such as 1,3-bis(2,6-diisopropylphenyl)-4,5-dihydro-1H-imidazolium chloride, 1,3-bis(2,6-diisopropylphenyl)imidazolium chloride, and 1,3-bis(2,4,6-trimethylphenyl)-4,5-dihydro-1H-imidazolium chloride; trialkylphosphonium tetrafluoroborate compounds such as t-Bu₃P·HBF₄ (tri-tert-butylphosphonium tetrafluoroborate); and the like.

When the heteroatom-containing compound is used in the method for producing a homocoupling reaction product according to the present invention, the amount thereof is not particularly limited, and is, for example, 10 mol or less, preferably 8 mol or less, more preferably 5 mol or less, still more preferably 3 mol or less, relative to 1 mol of the nickel catalyst. Using the heteroatom-containing compound enables the homocoupling reaction to proceed with high selectivity.

### <Reductant>

In the method for producing a homocoupling reaction product according to the present invention, a reductant can be further used. The reductant is not particularly limited as long as it can reduce divalent nickel to zero-valent nickel. Examples include compounds containing one or more selected from the group consisting of zinc, boron, manganese, magnesium, aluminum, sodium, and the like. Of these, one or more selected from the group consisting of zinc, boron, and manganese are preferred. These reductants can be used in any form, and can be used, for example, in the form of particles (fine particles, powder, chips, etc.) with any diameter.

Examples of the reductant in the method for producing a homocoupling reaction product according to the present invention include one or more selected from the group consisting of zinc powder, boron powder, manganese powder, magnesium powder, aluminum powder, sodium, sodium hydride, aluminum hydride compounds (e.g., lithium aluminum hydride, diisobutylaluminum hydride, and sodium bis(2-methoxyethoxy)aluminum hydride), borohydride compounds (e.g., sodium borohydride, lithium triethylborohydride, nickel borohydride, and zinc borohydride), silicon hydride compounds (e.g., triethylsilane and triisopropylsilane), tin hydride compounds (tributyltin hydride), and transition metal hydride compounds. Preferred are one or more selected from the group consisting of zinc powder, boron powder, manganese powder, magnesium powder, aluminum powder, and the like, and more preferred are one or more selected from the group consisting of zinc powder, boron powder, and manganese powder.

When the reductant is used in the method for producing a homocoupling reaction product according to the present invention, the amount thereof is not particularly limited, and is, for example, 100 mol or less, preferably 80 mol or less, more preferably 50 mol or less, still more preferably 30 mol or less, relative to 1 mol of the nickel catalyst. By using the reductant, the nickel catalyst in the reaction system is regenerated by the reductant, and the amount of the nickel catalyst used can be reduced.

### <Other components>

In the method for producing a homocoupling reaction product according to the present invention, other components can be further used in addition to the "organic halogen compound", the "nickel catalyst", the "solvent", the "heteroatom-containing compound", and the "reductant". As the other components, for example, other components such as a homocoupling reaction accelerator (e.g., water), a base, and an unsaturated hydrocarbon compound can be used.

### <Mechanochemical method>

The mechanochemical method used in the method for producing a homocoupling reaction product according to the present invention is a method in which mechanical energy is applied to the halogen compound (substrate), the nickel catalyst, and others serving as reaction components to cause a reaction. The mechanical energy can be mechanically generated by means of, for example, grinding, shearing, impacting, or compression. By applying such mechanical energy to the halogen compound, the nickel catalyst, and others, these can be activated to undergo a reaction. The mechanochemical reaction method is an organic synthesis reaction method in which components included in the reaction system are mixed in direct contact with each other to undergo a reaction and in which an organic solvent need not be used, and offers high reactivity while having a small burden on the environment.

The reaction device, the reaction conditions, etc. in the mechanochemical method used in the method for producing a homocoupling reaction product according to the present invention can be as follows.

### (Reaction device)

The reaction device used in the method for producing a homocoupling reaction product according to the present invention is not particularly limited as long as it is a device which can apply mechanical energy to components involved in the reaction and with which the homocoupling reaction can be carried out.

Examples of such devices include one or more selected from the group consisting of:
mills such as ball mills, rod mills, jet mills, and SAG mills;
grinders such as rotary millstones and grinding machines;
(horizontal-axis rotating) rotary vessel type mixing devices of horizontal cylinder type, V type, double cone type, cube type, S type, continuous V type, and other types;
rotary vessel type mixing devices (with baffle plates and blades) of horizontal cylinder type, V type, double cone type, ball mill type, and other types;
(rotationally oscillating) rotary vessel type mixing devices of rocking type, cross-rotary type, and other types;
(horizontal-axis rotating) fixed vessel type mixing devices of ribbon type, paddle type, single-axis rotor type, bug mill type, and other types;
(vertical-axis rotating) fixed vessel type mixing devices of ribbon type, screw type, planetary type, turbine type, high-speed flow type, rotating disk type, muller type, and other types;
(vibratory) fixed vessel type mixing devices of vibratory mill type, sieve type, and other types;
(fluidal) hydrokinetic mixing devices of heterogeneous fluidized bed type, swirling fluidized bed type, riser tube type, jet pump type, and other types;
(gravitational) hydrokinetic mixing devices of gravity type, static mixer type, and other types;
kneaders such as twin-screw kneaders, single-screw kneaders, mixers, and roll mills;
and the like.

In the method for producing a homocoupling reaction product according to the present invention, one or more selected from the group consisting of mills such as ball mills, grinders, mixing devices, kneaders, and the like are preferably used, and a mixing device is particularly preferably used. For the mixing device, for example, reference can be made to the powder mixing devices described in Table 5, Fig. 9, etc. of Sakashita "Mixture Process Technology for the Powders" Journal of the Japan Society of Colour Material, 77(2), 75-85 (2004). Specifically, a ball mill, a twin-screw kneader, a planetary ball mill, a SPEX mixer mill, a twin-screw ball mill, or the like can be used.

The device used in the reaction by the mechanochemical method may include one or more means selected from the group consisting of measuring means, depressurizing or pressurizing means, atmosphere adjusting means (gas introduction or discharge means), means for introducing various components, means for discharging various components and reaction products, purification means, analysis means, reaction monitoring means, and the like.

### (Reaction vessel)

In the method for producing a homocoupling reaction product according to the present invention, the reaction vessel used in the reaction by the mechanochemical method is not particularly limited as long as it is a reaction vessel in which the homocoupling reaction can be carried out in consideration of the physical properties, reactivity, amount, etc. of the organic halogen compound, the nickel catalyst, and other components present in the reaction system and the conditions, etc. of the reaction by the mechanochemical method. For example, when a device that performs mixing treatment in a mechanical manner (e.g., a ball mill) is used, a ball mill jar or the like can be used as the reaction vessel.

In the method for producing a homocoupling reaction product according to the present invention, the reaction vessel may include a means for stirring the components involved in the reaction in the reaction vessel. The means for stirring the components involved in the reaction in the reaction vessel is not particularly limited as long as it is a stirring means that can be provided in the reaction device. Means using the devices that perform mixing treatment in a mechanical manner described in (Reaction device) above can be used. As the device that performs mixing treatment in a mechanical manner, for example, a ball mill is preferably used.

### (Reaction conditions in mechanochemical method)

In the method for producing a homocoupling reaction product according to the present invention, the reaction conditions in the reaction by the mechanochemical method are not particularly limited, and are not particularly limited as long as they are reaction conditions where the homocoupling reaction can be carried out in consideration of the physical properties, reactivity, amount, etc. of the organic halogen compound, the nickel catalyst, and other components present in the reaction system and the conditions, etc. of the reaction by the mechanochemical method.

Although not clear, the principle and other details of the mechanochemical method are probably as follows.

Mechanical energy is applied to the components involved in the homocoupling reaction, and the components involved in the reaction absorb the mechanical energy, so that the surfaces of the components involved in the reaction are activated. As a result, the components involved in the reaction are activated, and a chemical reaction occurs between the surfaces having energy, leading to an intermolecular reaction. For example, when a ball mill is used, the surfaces of the components involved in the reaction (e.g., the halogen compound) are activated upon application of mechanical energy to the components involved in the reaction.

### (Mechanical energy to be applied)

In the method for producing a homocoupling reaction product according to the present invention, the mechanical energy applied in the reaction by the mechanochemical method is not particularly limited as long as it is mechanical energy with which the homocoupling reaction can be carried out.

For example, when a mixing device is used as the device used in the reaction by the mechanochemical method, the mixing rate is not particularly limited, and can be appropriately determined in consideration of the physical properties, reactivity, amount, etc. of the organic halogen compound, the nickel catalyst, and other components present in the reaction system and the conditions, etc. of the reaction by the mechanochemical method. For example, when a ball mill is used, shaking can be performed at 5 Hz or more, preferably 10 Hz or more, more preferably 20 Hz or more.

### (Reaction temperature)

In the method for producing a homocoupling reaction product according to the present invention, the reaction temperature (the temperature in the reaction vessel during mixing) is not particularly limited. A temperature at which the homocoupling reaction can be carried out can be employed in consideration of the physical properties, reactivity, amount, etc. of the organic halogen compound, the nickel catalyst, and other components present in the reaction system and the conditions, etc. of the reaction by the mechanochemical method. The reaction temperature is, for example, 20°C or higher, preferably 50°C or higher, more preferably 60°C or higher, and is, for example, 500°C or lower, preferably 300°C or lower, more preferably 250°C or lower.

The method of controlling the reaction temperature is not particularly limited, and a temperature control method used in performing a chemical reaction can be used. Examples include controlling the temperature in the reaction vessel by using warm air, controlling the temperature in the reaction vessel by covering the reaction vessel with a heating medium at a predetermined temperature, and controlling the temperature in the reaction vessel by providing a heating element. Of these, controlling the temperature in the reaction vessel by applying warm air generated with a heat gun to the reaction vessel is preferred, for example, from the viewpoint of safety and ease of temperature control operation.

### (Pressure)

In the method for producing a homocoupling reaction product according to the present invention, the reaction pressure in the reaction by the mechanochemical method (the pressure in the reaction vessel during mixing) is not particularly limited. A pressure at which the homocoupling reaction can be carried out can be employed in consideration of the physical properties, reactivity, amount, etc. of the organic halogen compound, the nickel catalyst, and other components present in the reaction system and the conditions, etc. of the reaction by the mechanochemical method. If necessary, the reaction pressure can be controlled using a decompression device or a pressurization device, and the reaction can be carried out without pressurization or decompression. Of these, it is preferable to carry out the reaction without pressurization or decompression (carry out the reaction under atmospheric pressure), for example, from the viewpoint of the reaction operation, the reaction device, etc.

### (Reaction atmosphere)

In the method for producing a homocoupling reaction product according to the present invention, the reaction atmosphere (the atmosphere in the reaction vessel during mixing) is not particularly limited. A reaction atmosphere in which the homocoupling reaction can be carried out can be employed in consideration of the physical properties, reactivity, amount, etc. of the organic halogen compound, the nickel catalyst, and other components present in the reaction system and the conditions, etc. of the reaction by the mechanochemical method.

For example, the reaction can be carried out in an air atmosphere without any particular atmosphere adjustment. If necessary, the reaction can be carried out in an inert gas atmosphere such as nitrogen, helium, neon, or argon.

For example, bis(1,5-cyclooctadiene)nickel(0) [Ni(cod)₂], which is widely used as a nickel catalyst, is unstable in air and needs to be handled and used in an inert gas atmosphere, but in the homocoupling method according to the present invention, it can be used without adjusting the atmosphere in the reaction vessel.

### (Reaction time)

In the method for producing a homocoupling reaction product according to the present invention, the reaction time (mixing time; the time period during which treatment by mechanical means is performed) is not particularly limited. The reaction time can be appropriately determined in consideration of the physical properties, reactivity, amount, etc. of the organic halogen compound, the nickel catalyst, and other components present in the reaction system and the conditions, etc. of the reaction by the mechanochemical method.

For example, the reaction time can be 1 minute or more, preferably 3 minutes or more, more preferably 5 minutes or more. The upper limit of the reaction time is not particularly limited, and can be, for example, 10 hours or less, preferably 5 hours or less, more preferably 3 hours or less.

### (Input order of reaction components, treatment after reaction, etc.)

In the method for producing a homocoupling reaction product according to the present invention, the order in which the reaction components are put in the reaction vessel in the reaction by the mechanochemical method is not particularly limited. The means of input is also not particularly limited.

In the method for producing a homocoupling reaction product according to the present invention, a multistep synthesis reaction can be employed in which the preceding reaction is carried out by the mechanochemical method, after which the reaction product is isolated, and then reaction components including the preceding reaction product are put in the reaction vessel again to perform subsequent reactions.

In the method for producing a homocoupling reaction product according to the present invention, a one-pot sequential reaction can be employed in which the preceding reaction is carried out by the mechanochemical method, after which without isolating the reaction product and others, reaction components necessary for the subsequent reaction are put in the reaction vessel in which the preceding reaction has been finished to perform subsequent reactions.

After completion of the reaction, the resulting homocoupling reaction product may be purified as needed.

The purification method is not particularly limited, and, for example, a method such as filtration, distillation, recrystallization, column chromatography, or washing with a solvent is used.

### [Reaction product of homocoupling reaction]

In the method for producing a homocoupling reaction product according to the present invention, examples of the reaction product of the homocoupling reaction in the case where a compound represented by formula (I);

A¹-Xₘ (I)

(In formula (I), A¹, X, and m are the same as described in [Method for producing homocoupling reaction product] above.) is used as the organic halogen compound include the following.
(1) When an organic halogen compound in the case where m = 1 (a monohalogen compound) is used, examples include homocoupling reaction products represented by formula (A);

   A¹-A¹ (A)

   (In formula (A), A¹ may be identical or different.).
(2) When an organic halogen compound in the case where m = 2 (a dihalogen compound) is used, examples include linear or cyclic homocoupling reaction polymerization products and intramolecular homocoupling reactions having a repeating unit represented by formula (P1); 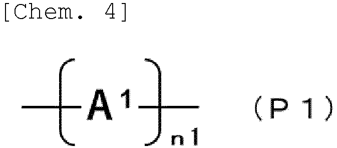 (In formula (P1), a plurality of A¹'s may be identical or different, and n1 is an integer of 2 or more.).
(3) When an organic halogen compound in the case where m ≥ 3 (e.g., a trihalogen compound or a tetrahalogen compound) is used, examples include homocoupling reaction crosslinking polymerization products and intramolecular homocoupling reaction products.

Examples of homocoupling reaction crosslinking polymerization products in the case where m = 3 include polymers having a unit represented by formula (P2); 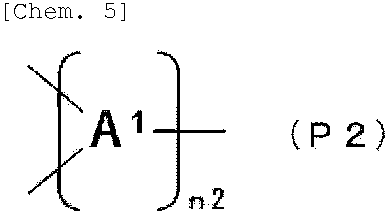 (In formula (P2), a plurality of A¹'s may be identical or different, and n2 is an integer of 2 or more.).

Examples of homocoupling reaction crosslinking polymerization products in the case where m = 4 include polymers having a unit represented by formula (P3); 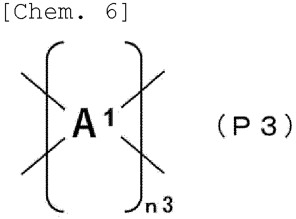 (In formula (P3), a plurality of A¹'s may be identical or different, and n3 is an integer of 2 or more.).

The reaction products obtained by the method for producing a homocoupling reaction product according to the present invention may be reaction products having various structures other than the reaction products exemplified as formula (A) and formula (P1) to formula (P3) above.

The reaction products obtained by the method for producing a homocoupling reaction product according to the present invention can be used as components of, for example, functional materials such as pharmaceuticals, liquid crystal compounds, organic electroluminescence compounds, organic thin-film solar cells, macromolecular compounds, oligomers, coloring materials, radiation absorbing materials, information recording materials, wavelength conversion materials, indicator materials, sensor materials, organic light-emitting diodes (OLEDs), and organic semiconductor materials.

### EXAMPLES

The present invention will now be described in detail with reference to specific examples. These specific examples are merely embodiments of the present invention. The present invention is not limited at all by these examples.

"Equiv" denotes "equivalent".

In Examples and Comparative Examples, when a reaction was carried out using a ball mill, reagents were put in a stainless-steel ball mill jar, and an MM400 model ball mill manufactured by Verder Scientific Co., Ltd. (formerly Retsch) was used.

When a homocoupling reaction was carried out using a ball mill, heating was performed such that the outside of a ball mill jar was heated at a predetermined temperature with a heat gun (HG-1450B manufactured by Takagi Co., Ltd.).

The relationship between the heat gun set temperature and the ball mill jar internal temperature (the temperature in the reaction system) was examined using a thermographic image and found to be as follows.

**[Table 1]**

| Heat gun temperature | Jar internal temperature |
|---|---|
| 300°C | 125°C |
| 250°C | 120°C |
| 200°C | 100°C |
| 160°C | 80°C |
| 150°C | 74°C |
| No Heating | 32°C |

### [Homocoupling reaction of monohalogen compound by mechanochemical method]

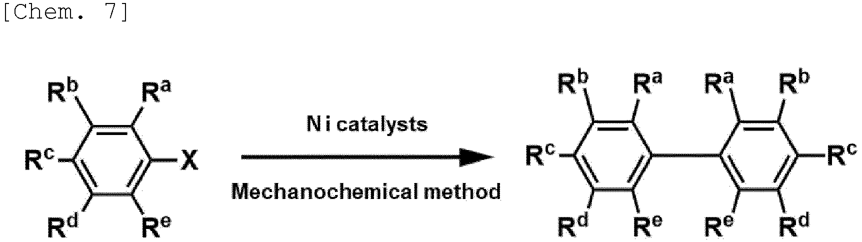

### {Study of mechanochemical reaction conditions}

### <Example 1>

To a 5 mL stainless-steel ball mill jar containing stainless-steel balls having a diameter of 10 mm, 0.2 mmol of 1-bromo-3,5-dimethoxybenzene (A-1) as a monohalogen compound and 1.0 equiv of 2,2'-bipyridine (C-1; melting point, 70°C to 72°C) as a heteroatom-containing compound were added under air. Next, the ball mill jar was placed in a glove box filled with argon, and 1.0 equiv of bis(1,5-cyclooctadiene)nickel(0) as a nickel catalyst was added to the ball mill jar. The ball mill jar was capped, taken out from the glove box, and mounted to a ball mill. While heating was performed at a heat gun set temperature of 160°C (a ball mill jar internal temperature of 80°C), shaking and stirring were performed at a frequency of 30 Hz for 10 minutes to cause a reaction.

Thereafter, the reaction product was extracted, dried, and purified, thus obtaining a homocoupling reaction product (B-1) via the reaction by the mechanochemical method. The NMR yield was 56%.

### <Examples 2 to 5>

The homocoupling reaction was carried out in the same manner as in Example 1 except that the frequency and the time were changed as shown in Table 2 to obtain a homocoupling reaction product (B-1). The NMR yield is also shown in Table 2.

**[Table 2]**

| Example | Frequency (Hz) | Time (min) | NMR yield |
|---|---|---|---|
| 1 | 30 | 10 | 56% |
| 2 | 30 | 30 | 54% |
| 3 | 25 | 30 | 58% |
| 4 | 20 | 30 | 71% |
| 5 | 20 | 10 | 68% |

### {Study of heteroatom-containing compound}

### <Example 6>

To a 5 mL stainless-steel ball mill jar containing stainless-steel balls having a diameter of 10 mm, 0.2 mmol of 1-bromo-3,5-dimethoxybenzene (A-1) as a monohalogen compound and 1.0 equiv of 2,2'-bipyridine (C-1; melting point, 70°C to 72°C) as a heteroatom-containing compound were added under air. Next, the ball mill jar was placed in a glove box filled with argon, and 1.0 equiv of bis(1,5-cyclooctadiene)nickel(0) as a nickel catalyst was added to the ball mill jar. The ball mill jar was capped, taken out from the glove box, and mounted to a ball mill. While heating was performed at a heat gun set temperature of 160°C (a ball mill jar internal temperature of 80°C), shaking and stirring were performed at a frequency of 20 Hz for 30 minutes to cause a reaction.

Thereafter, the reaction product was extracted, dried, and purified, thus obtaining a homocoupling reaction product (B-1) via the reaction by the mechanochemical method. The NMR yield was 71%.

### <Examples 7 to 13>

The homocoupling reaction was carried out in the same manner as in Example 6 except that (C-2) to (C-8) shown in Table 3 were used as heteroatom-containing compounds to obtain a homocoupling reaction product (B-1). The NMR yield is also shown in Table 3.

**[Table 3]**

| Example | Heteroatom-containing compound | NMR yield |
|---|---|---|
| 6 | C-1 | 71% |
| 7 | C-2 | 86% |
| 8 | C-3 | 80% |
| 9 | C-4 | 48% |
| 10 | C-5 | 58% |
| 11 | C-6 | 46% |
| 12 | C-7 | 43% |
| 13 | C-8 | 65% |

### {Study of temperature and reaction scale}

### <Example 14>

To a 5 mL stainless-steel ball mill jar containing stainless-steel balls having a diameter of 10 mm, 0.20 mmol of 1-bromo-3,5-dimethoxybenzene (A-1) as a monohalogen compound and 1.0 equiv (0.20 mmol) of 4,4'-bis(di-t-butyl)-2,2'-bipyridine (C-2; melting point, 161°C) as a heteroatom-containing compound were added under air. Next, the ball mill jar was placed in a glove box filled with argon, and 1.0 equiv (0.20 mmol) of bis(1,5-cyclooctadiene)nickel(0) as a nickel catalyst was added to the ball mill jar. The ball mill jar was capped, taken out from the glove box, and mounted to a ball mill. While heating was performed at a heat gun set temperature of 160°C (a ball mill jar internal temperature of 80°C), shaking and stirring were performed at a frequency of 20 Hz for 30 minutes to cause a reaction.

Thereafter, the reaction product was extracted, dried, and purified, thus obtaining a homocoupling reaction product (B-1) via the reaction by the mechanochemical method. The NMR yield was 86%.

### <Examples 15 and 16>

The homocoupling reaction was carried out in the same manner as in Example 14 except that the amount of the monohalogen compound used and the heat gun set temperature (ball mill jar internal temperature) were changed as shown in Table 4 to obtain a homocoupling reaction product (B-1). The NMR yield is also shown in Table 4.

### <Example 17>

The homocoupling reaction was carried out in the same manner as in Example 14 except that the amount of the nickel catalyst used was 1.2 equiv, the amount of the heteroatom-containing compound used was 1.2 equiv, and the amount of the monohalogen compound used and the heat gun set temperature (ball mill jar internal temperature) were changed as shown in Table 4 to obtain a homocoupling reaction product (B-1). The NMR yield is also shown in Table 4.

**[Table 4]**

| Example | Amount of monohalogen compound used | Heat gun set temperature | Ball mill jar internal temperature | NMR yield |
|---|---|---|---|---|
| 14 | 0.2 mmol | 160°C | 80°C | 86% |
| 15 | 0.2 mmol | 250°C | 120°C | 91% |
| 16 | 0.5 mmol | 250°C | 120°C | 80% |
| 17 | 0.5 mmol | 250°C | 120°C | 93% |

### {Study of monohalogen compound}

### <Example 18>

To a 5 mL stainless-steel ball mill jar containing stainless-steel balls having a diameter of 10 mm, 0.5 mmol of 1-bromo-3,5-dimethoxybenzene (A-1) as a monohalogen compound and 1.2 equiv of 4,4'-bis(di-t-butyl)-2,2'-bipyridine (C-2; melting point, 161°C) as a heteroatom-containing compound were added under air. Next, the ball mill jar was placed in a glove box filled with argon, and 1.2 equiv of bis(1,5-cyclooctadiene)nickel(0) as a nickel catalyst was added to the ball mill jar. The ball mill jar was capped, taken out from the glove box, and mounted to a ball mill. While heating was performed at a heat gun set temperature of 200°C (a ball mill jar internal temperature of 100°C), shaking and stirring were performed at a frequency of 20 Hz for 30 minutes to cause a reaction.

Thereafter, the reaction product was extracted, dried, and purified, thus obtaining a homocoupling reaction product (B-1) via the reaction by the mechanochemical method. The isolated yield was 91%.

### <Examples 19 to 21>

The homocoupling reaction was carried out in the same manner as in Example 18 except that compounds shown in Table 5 were used as monohalogen compounds to obtain corresponding homocoupling reaction products shown in Table 5. The isolated yield is also shown in Table 5.

**[Table 5]**

| Example | Monohalogen compound | Homocoupling reaction product | Isolated yield | Heat gun set temperature | Shaking and stirring conditions (time/frequency) |
|---|---|---|---|---|---|
| 18 | A-1 | B-1 | 91% | 200°C | 30 min/20 Hz |
| 19 | A-2 | B-2 | 95% | 200°C | 30 min/20 Hz |
| 20 | A-3 | B-3 | 85% | 200°C | 30 min/20 Hz |
| 21 | A-4 | B-4 | 50% | 200°C | 30 min/20 Hz |

### <Example 22>

To a 1.5 mL stainless-steel ball mill jar containing stainless-steel balls having a diameter of 5 mm, 0.2 mmol of 1-bromo-3,5-dimethoxybenzene (A-1) as a monohalogen compound and 1.0 equiv of phenanthroline (C-4; melting point, 117°C) as a heteroatom-containing compound were added under air. Next, the ball mill jar was placed in a glove box filled with argon, and 1.0 equiv of bis(1,5-cyclooctadiene)nickel(0) as a nickel catalyst was added to the ball mill jar. The ball mill jar was capped, taken out from the glove box, and mounted to a ball mill. While heating was performed at a heat gun set temperature of 160°C (a ball mill jar internal temperature of 80°C), shaking and stirring were performed at a frequency of 30 Hz for 10 minutes to cause a reaction.

Thereafter, the reaction product was extracted, dried, and purified, thus obtaining a homocoupling reaction product (B-1) via the reaction by the mechanochemical method. The isolated yield was 99%.

### <Examples 23 to 28>

The homocoupling reaction was carried out in the same manner as in Example 22 except that compounds shown in Table 6 were used as monohalogen compounds, 2,2'-bipyridine (C-1: melting point, 70°C to 72°C) was used as a heteroatom-containing compound, and the shaking and stirring conditions were changed as shown in Table 6 to obtain corresponding reaction products shown in Table 6. The isolated yield is also shown in Table 6.

**[Table 6]**

| Example | Monohalogen compound | Homocoupling reaction product | Isolated yield | Shaking and stirring conditions (time/ frequency) |
|---|---|---|---|---|
| 22 | A-1 | B-1 | 99% | 10 min/30 Hz |
| 23 | A-5 | B-5 | 70% | 30 min/30 Hz |
| 24 | A-6 | B-6 | 67% | 30 min/30 Hz |
| 25 | A-2 | B-2 | 66% | 10 min/30 Hz |
| 26 | A-7 | B-7 | 51% | 30 min/30 Hz |
| 27 | A-8 | B-8 | 66% | 10 min/30 Hz |
| 28 | A-9 | B-9 | 91% | 60 min/30 Hz |

### <Example 29>

To a 5 mL stainless-steel ball mill jar containing stainless-steel balls having a diameter of 10 mm, 0.5 mmol of N,N-diphenyl-4-bromoaniline (A-5) as a monohalogen compound and 1.2 equiv (0.6 mmol) of 4,4'-bis(di-t-butyl)-2,2'-bipyridine (C-2; melting point, 161°C) as a heteroatom-containing compound were added under air. Next, the ball mill jar was placed in a glove box filled with argon, and 1.2 equiv (0.6 mmol) of bis(1,5-cyclooctadiene)nickel(0) as a nickel catalyst was added to the ball mill jar. The ball mill jar was capped, taken out from the glove box, and mounted to a ball mill. While heating was performed at a heat gun set temperature of 250°C (a ball mill jar internal temperature of 120°C), shaking and stirring were performed at a frequency of 20 Hz for 30 minutes to cause a reaction.

Thereafter, the reaction product was extracted, dried, and purified, thus obtaining a homocoupling reaction product (B-5) via the reaction by the mechanochemical method. The isolated yield was 99%.

### <Examples 30 to 41>

The homocoupling reaction was carried out in the same manner as in Example 29 except that compounds shown in Table 7 were used as monohalogen compounds to obtain corresponding reaction products shown in Table 7. The isolated yield is also shown in Table 7.

**[Table 7]**

| Example | Monohalogen compound | Homocoupling reaction product | Isolated yield |
|---|---|---|---|
| 29 | A-5 | B-5 | 99% |
| 30 | A-10 | B-10 | 97% |
| 31 | A-11 | B-11 | 73% |
| 32 | A-3 | B-3 | 85% |
| 33 | A-12 | B-2 | 95% |
| 34 | A-13 | B-12 | 63% |
| 35 | A-14 | B-13 | 99% |
| 36 | A-6 | B-6 | 89% |
| 37 | A-15 | B-14 | 98% |
| 38 | A-4 | B-4 | 50% |
| 39 | A-16 | B-15 | 82% |
| 40 | A-17 | B-16 | 89% |
| 41 | A-18 | B-17 | 93% |

### {Study of reactivity of halogen species}

### <Example 42>

To a 5 mL stainless-steel ball mill jar containing stainless-steel balls having a diameter of 10 mm, 0.5 mmol of methyl 4-bromobenzoate (A-3) as a monohalogen compound, 1.2 equiv (0.6 mmol) of bis(1,5-cyclooctadiene)nickel(0) as a nickel catalyst, and 1.2 equiv (0.6 mmol) of 4,4'-bis(dit-butyl)-2,2'-bipyridine (C-2; melting point, 161°C) as a heteroatom-containing compound were added under air. The ball mill jar was capped, taken out from the glove box, and mounted to a ball mill. While heating was performed at a heat gun set temperature of 250°C (a ball mill jar internal temperature of 120°C), shaking and stirring were performed at a frequency of 20 Hz for 30 minutes to cause a reaction.

Thereafter, the reaction product was extracted, dried, and purified, thus obtaining a homocoupling reaction product (B-3) via the reaction by the mechanochemical method. The NMR yield was 83%.

### <Examples 43 and 44>

The homocoupling reaction was carried out in the same manner as in Example 42 except that methyl 4-iodobenzoate (A-19) was used as a monohalogen compound in Example 43 and methyl 4-chlorobenzoate (A-20) was used as a monohalogen compound in Example 44 to obtain a homocoupling reaction product (B-3). The NMR yield is also shown in Table 8.

**[Table 8]**

| Example | Monohalogen compound | Homocoupling reaction product | NMR yield |
|---|---|---|---|
| 42 | A-3 | B-3 | 83% |
| 43 | A-19 | B-3 | 81% |
| 44 | A-20 | B-3 | 87% |

### [Scale-up of homocoupling reaction]

### <Example 45>

To a 10 mL stainless-steel ball mill jar containing stainless-steel balls having a diameter of 10 mm, 4.2 mmol of N,N-diphenyl-4-bromoaniline (A-5) as a monohalogen compound, 1.2 equiv of bis(1,5-cyclooctadiene)nickel(0) as a nickel catalyst, and 1.2 equiv of 4,4'-bis(di-t-butyl)-2,2'-bipyridine (C-2; melting point, 161°C) as a heteroatom-containing compound were added under air. The ball mill jar was capped, taken out from the glove box, and mounted to a ball mill. While heating was performed at a heat gun set temperature of 250°C (a ball mill jar internal temperature of 120°C), shaking and stirring were performed at 20 Hz for 30 minutes to cause a reaction.

Thereafter, the reaction product was extracted, dried, and purified, thus obtaining a homocoupling reaction product (B-5) via the reaction by the mechanochemical method. The isolated yield was 99% (1.026 g).

### [Monohalogen compound, homocoupling reaction product, and heteroatom-containing compound]

Monohalogen compounds (A-1) to (A-20), homocoupling reaction products (B-1) to (B-17), and heteroatom-containing compounds (C-1) to (C-8) used in Examples 1 to 45 are as follows. In these structural formulas, t-Bu denotes a tertiary butyl group.

### {Monohalogen compound}

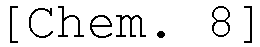

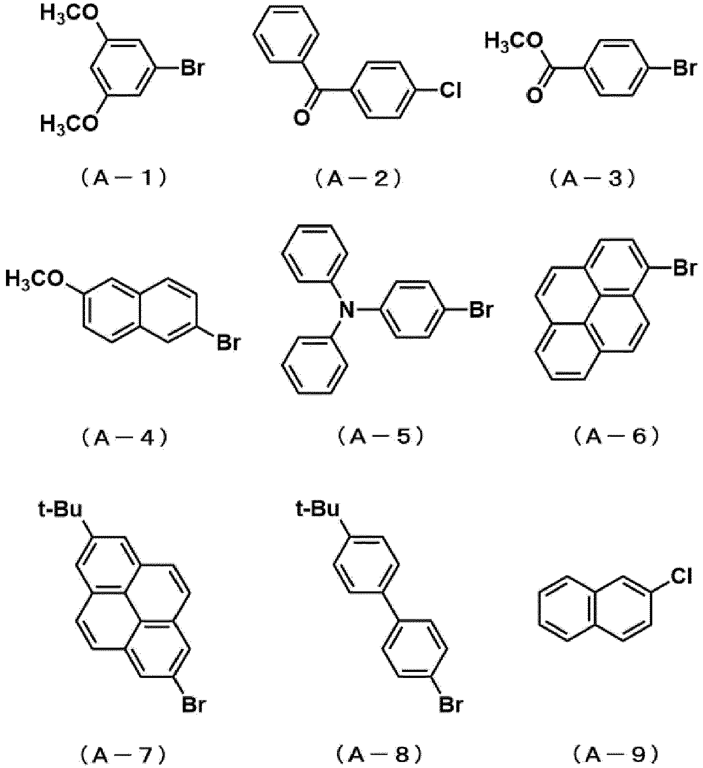

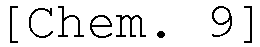

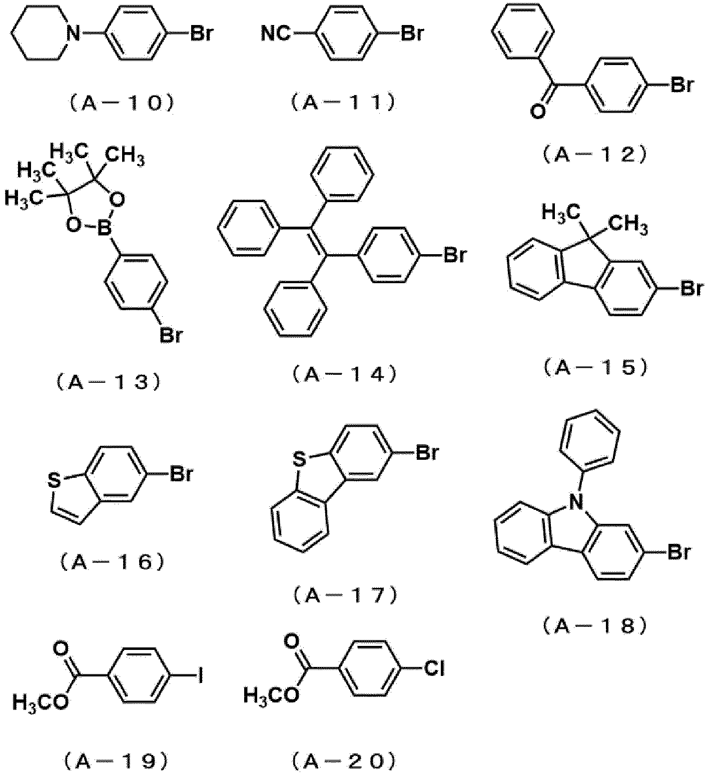

### {Homocoupling reaction product}

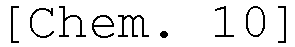

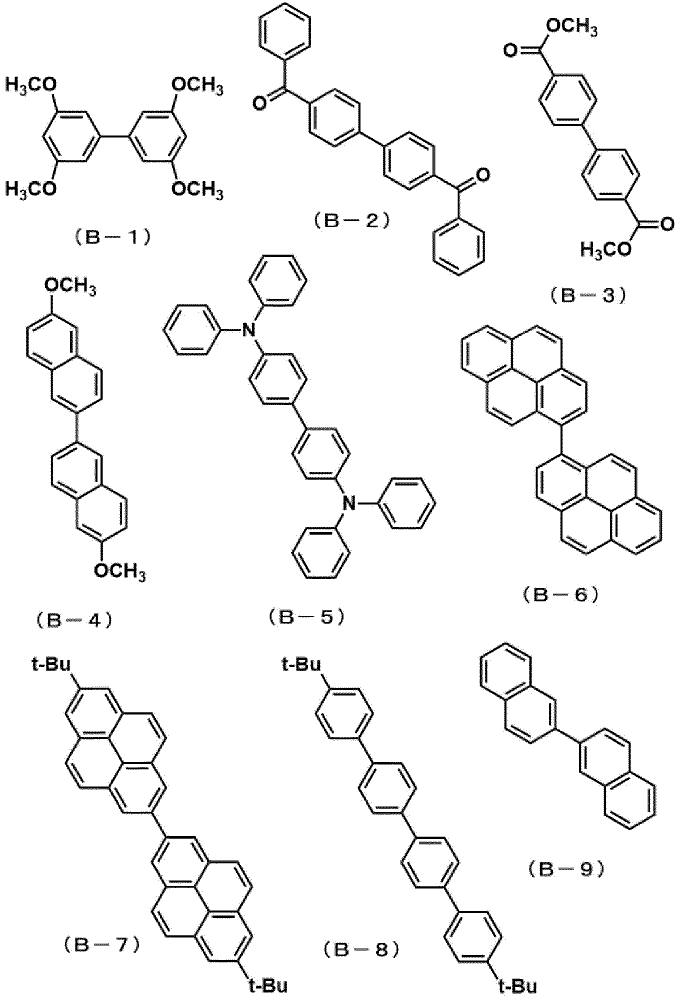

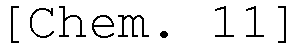

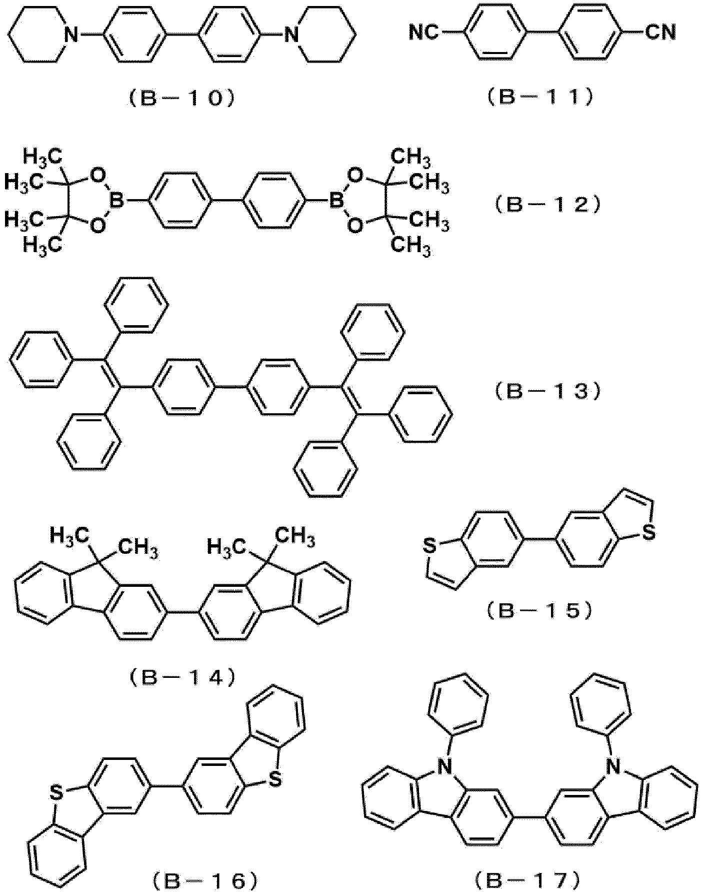

### {Heteroatom-containing compound}

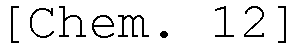

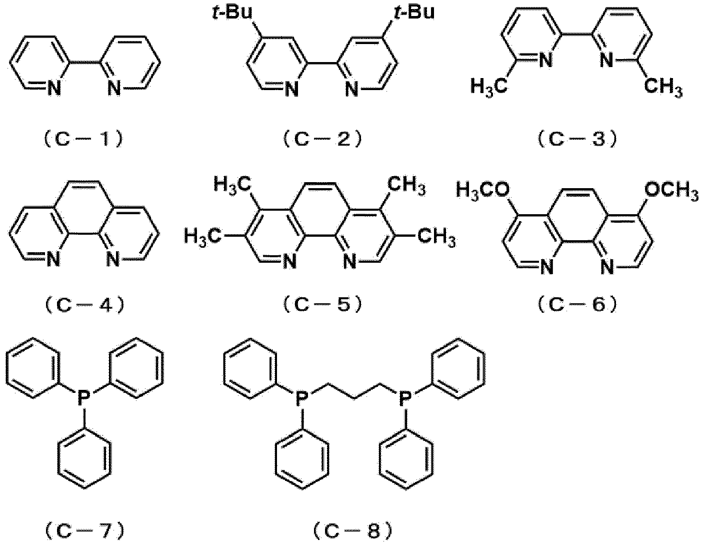

### [Homocoupling reaction in presence of reductant]

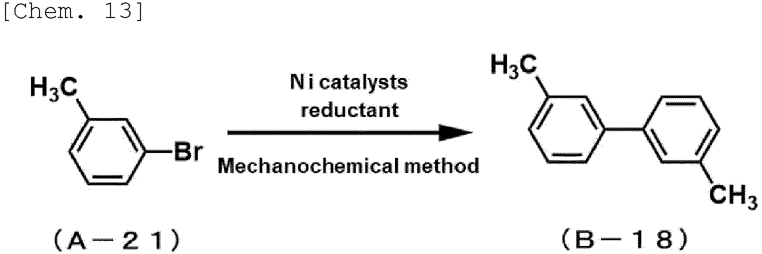

### <Example 46>

To a 1.5 mL stainless-steel ball mill jar containing stainless-steel balls having a diameter of 5 mm, 0.5 mmol of 3-bromotoluene (A-21) as a monohalogen compound, 0.05 mmol of bis(triphenylphosphine)nickel(II) dichloride as a nickel catalyst, 1.0 mmol of metal zinc dust as a reductant, and 60 µL of tetrahydrofuran (THF: melting point, -108°C) as a solvent were added under air. The ball mill jar was capped, taken out from the glove box, and mounted to a ball mill. While heating was performed at a heat gun set temperature of 250°C (a ball mill jar internal temperature of 120°C), shaking and stirring were performed at 30 Hz for 60 minutes to cause a reaction.

Thereafter, the reaction product was extracted, dried, and purified, thus obtaining a homocoupling reaction product (B-18) via the reaction by the mechanochemical method. The gas chromatography yield was 44%.

### [Homocoupling reaction of 1,2-dibromobenzene]

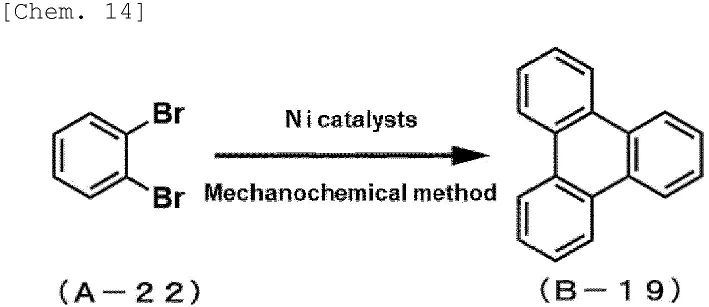

### <Example 47>

To a 5 mL stainless-steel ball mill jar containing stainless-steel balls having a diameter of 10 mm, 0.5 mmol of 1,2-dibromobenzene (A-22) as a halogen compound, 2.4 equiv of bis(1,5-cyclooctadiene)nickel(0) as a nickel catalyst, and 2.4 equiv of 4,4'-bis(di-t-butyl)-2,2'-bipyridine as a heteroatom-containing compound were added under air. The ball mill jar was capped, taken out from the glove box, and mounted to a ball mill. While heating was performed at a heat gun set temperature of 200°C (a ball mill jar internal temperature of 100°C), shaking and stirring were performed at 20 Hz for 30 minutes to cause a reaction.

Thereafter, the reaction product was extracted, dried, and purified, thus obtaining a homocoupling reaction product (B-19) via the reaction by the mechanochemical method. The NMR yield was 26%.

[Application of homocoupling reaction by mechanochemical method to polymerization]
{Homocoupling polymerization reaction of 1,4-dibromo-2,5-bis(decyloxy)benzene}

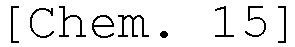

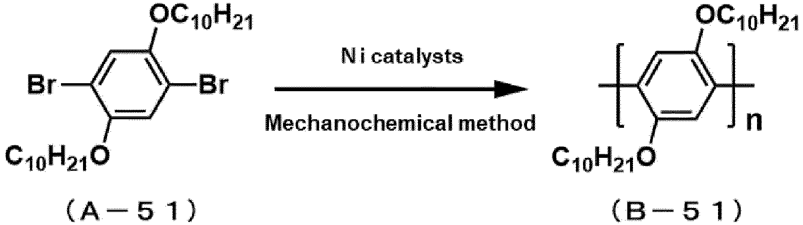

### <Example 48>

To a 1.5 mL stainless-steel ball mill jar containing stainless-steel balls having a diameter of 5 mm, 56.6 mg (0.1 mmol) of 1,4-dibromo-2,5-bis(decyloxy)benzene (A-51) as a halogen compound and 32.3 mg (0.2 mmol) of 2,2'-bipyridyl as a heteroatom-containing compound were added under air. Thereafter, the ball mill jar was transferred to a glove box, and 55.1 mg (0.2 mmol) of bis(1,5-cyclooctadiene)nickel(0) as a nickel catalyst was added.

The ball mill jar was taken out from the glove box, and the ball mill jar was capped and mounted to a ball mill. While heating was performed at a heat gun set temperature of 160°C (a ball mill jar internal temperature of 80°C), shaking and stirring were performed at 30 Hz for 10 minutes to cause a reaction.

After completion of the reaction, the reaction product was extracted using dichloromethane and water, and an organic layer was dried over magnesium sulfate. After filtration, dichloromethane was removed with an evaporator. The crude product was reprecipitated in a dichloromethane/methanol solvent to thereby obtain 33.1 mg of a homocoupling polymerization reaction product. The homocoupling polymerization reaction product was a polymer having a repeating unit represented by (B-51). The conversion rate was 95% or more, and the isolated yield was 87%. The number-average molecular weight (Mn), weight-average molecular weight (Mw), and polydispersity index (PDI) of the reaction product as determined by size exclusion chromatography (SEC) were as follows.
· Mn = 4.3 kg/mol
· Mw = 5.5 kg/mol
· PDI = 1.28

### <Example 49>

To a 1.5 mL stainless-steel ball mill jar containing stainless-steel balls having a diameter of 5 mm, 0.3 mmol of 1,4-dibromo-2,5-bis(decyloxy)benzene (A-51) as a halogen compound and 2.4 equiv (0.72 mmol), relative to the halogen compound, of 4,4'-bis(di-t-butyl)-2,2'-bipyridine as a heteroatom-containing compound were added under air. Thereafter, the ball mill jar was transferred to a glove box, and 2.4 equiv (0.72 mmol), relative to the halogen compound, of bis(1,5-cyclooctadiene)nickel(0) as a nickel catalyst was added. The ball mill jar was taken out from the glove box, and the ball mill jar was capped and mounted to a ball mill. While heating was performed at a heat gun set temperature of 200°C (a ball mill jar internal temperature of 100°C), shaking and stirring were performed at 20 Hz for 30 minutes to cause a reaction.

After completion of the reaction, the reaction product was extracted using dichloromethane and water, and an organic layer was dried over magnesium sulfate. After filtration, dichloromethane was removed with an evaporator. The crude product was reprecipitated in a dichloromethane/methanol solvent to thereby obtain a homocoupling polymerization reaction product. The homocoupling polymerization reaction product was a polymer having a repeating unit represented by (B-51), and the isolated yield was 70%. The number-average molecular weight (Mn), weight-average molecular weight (Mw), and polydispersity index (PDI) of the reaction product as determined by size exclusion chromatography (SEC) were as follows, and it was found that a 22-mer was mainly obtained.
· Mn = 8.6 kg/mol
· Mw = 11.4 kg/mol
· PDI = 1.33

### <Comparative Example 1>

To a reaction vessel, 0.3 mmol of 1,4-dibromo-2,5-bis(decyloxy)benzene (A-51) as a halogen compound, 2.4 equiv (0.72 mmol), relative to the halogen compound, of **4,4'-**bis(di-t-butyl)-2,2'-bipyridine as a heteroatom-containing compound, 2.4 equiv (0.72 mmol), relative to the halogen compound, of bis(1,5-cyclooctadiene)nickel(0) as a nickel catalyst, and 3 mL (0.1 M based on the halogen compound) of N,N-dimethylformamide (DMF) as a solvent were added, and a reaction was carried out in the solution at 80°C for 24 hours.

After completion of the reaction, the reaction product was extracted using dichloromethane and water, and an organic layer was dried over magnesium sulfate. After filtration, dichloromethane was removed with an evaporator. The crude product was reprecipitated in a dichloromethane/methanol solvent to thereby obtain a homocoupling polymerization reaction product. The homocoupling polymerization reaction product was a polymer having a repeating unit represented by (B-51), and the isolated yield was 51%. The number-average molecular weight (Mn), weight-average molecular weight (Mw), and polydispersity index (PDI) of the reaction product as determined by size exclusion chromatography (SEC) were as follows, and it was found that a 6.4-mer was mainly obtained.
· Mn = 2.5 kg/mol
· Mw = 2.9 kg/mol
. PDI = 1.17

### {Homocoupling polymerization reaction of 2,7-dibromo-9,9-bis(2-ethylhexyl)fluorene}

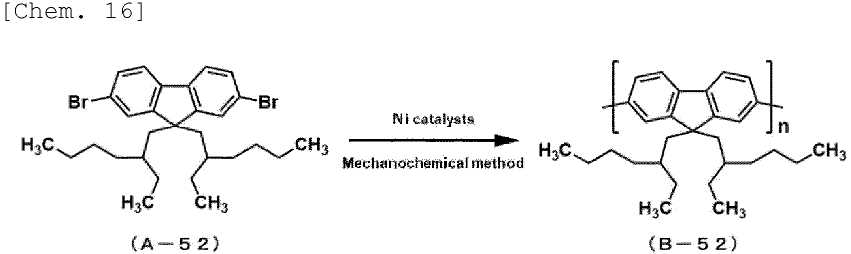

### <Example 50>

To a 1.5 mL stainless-steel ball mill jar containing stainless-steel balls having a diameter of 5 mm, 0.3 mmol of 2,7-dibromo-9,9-bis(2-ethylhexyl)fluorene (A-52) as a halogen compound and 2.4 equiv (0.72 mmol), relative to the halogen compound, of 4,4'-bis(di-t-butyl)-2,2'-bipyridine as a heteroatom-containing compound were added under air. Thereafter, the ball mill jar was transferred to a glove box, and 2.4 equiv (0.72 mmol), relative to the halogen compound, of bis(1,5-cyclooctadiene)nickel(0) as a nickel catalyst was added. The ball mill jar was taken out from the glove box, and the ball mill jar was capped and mounted to a ball mill. While heating was performed at a heat gun set temperature of 250°C (a ball mill jar internal temperature of 120°C), shaking and stirring were performed at 20 Hz for 30 minutes to cause a reaction.

After completion of the reaction, the reaction product was extracted using dichloromethane and water, and an organic layer was dried over magnesium sulfate. After filtration, dichloromethane was removed with an evaporator. The crude product was reprecipitated in a dichloromethane/methanol solvent to thereby obtain a homocoupling polymerization reaction product. The homocoupling polymerization reaction product was a polymer having a repeating unit represented by (B-52). The isolated yield was 85%. The number-average molecular weight (Mn), weight-average molecular weight (Mw), and polydispersity index (PDI) of the reaction product as determined by size exclusion chromatography (SEC) were as follows, and it was found that a 7.5-mer was mainly obtained.
· Mn = 2.9 kg/mol
· Mw = 9.7 kg/mol
· PDI = 3.25

### <Comparative Example 2>

To a reaction vessel, 0.3 mmol of 2,7-dibromo-9,9-bis(2-ethylhexyl)fluorene (A-22) as a halogen compound, 2.4 equiv (0.72 mmol), relative to the halogen compound, of 4,4'-bis(di-t-butyl)-2,2'-bipyridine as a heteroatom-containing compound, 2.4 equiv (0.72 mmol), relative to the halogen compound, of bis(1,5-cyclooctadiene)nickel(0) as a nickel catalyst, and 3 mL (0.1 M based on the halogen compound) of N,N-dimethylformamide (DMF) as a solvent were added, and a reaction was carried out in the solution at 80°C for 24 hours.

After completion of the reaction, the reaction product was extracted using dichloromethane and water, and an organic layer was dried over magnesium sulfate. After filtration, dichloromethane was removed with an evaporator. The crude product was reprecipitated in a dichloromethane/methanol solvent to thereby obtain a homocoupling polymerization reaction product. The homocoupling polymerization reaction product was a polymer having a repeating unit represented by (B-22). The isolated yield was 35%. The number-average molecular weight (Mn), weight-average molecular weight (Mw), and polydispersity index (PDI) of the reaction product as determined by size exclusion chromatography (SEC) were as follows, and it was found that an 8-mer was mainly obtained.
· Mn = 3.1 kg/mol
· Mw = 5.7 kg/mol
· PDI = 1.83

### {Homocoupling polymerization reaction of tetrakis(4-bromophenyl)ethylene}

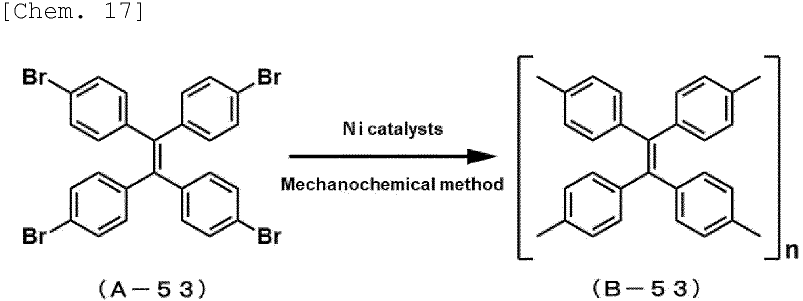

### <Example 51>

To a 5 mL stainless-steel ball mill jar containing stainless-steel balls having a diameter of 10 mm, 32.4 mg (0.05 mmol) of tetrakis(4-bromophenyl)ethylene (A-53) as a halogen compound, 55.0 mg (0.2 mmol) of bis(1,5-cyclooctadiene)nickel(0) as a nickel catalyst, and 31.2 mg (0.2 mmol) of 2,2'-bipyridine as a heteroatom-containing compound were added under air. The ball mill jar was capped and mounted to a ball mill. While heating was performed at a heat gun set temperature of 160°C (a ball mill jar internal temperature of 80°C), shaking and stirring were performed at 30 Hz for 10 minutes to cause a reaction.

Thereafter, the reaction product was extracted, dried, and purified to obtain 14.4 mg of a homocoupling polymerization reaction product. The homocoupling polymerization reaction product was a polymer having a unit with four bonds represented by (B-53). The isolated yield was 88%.

### {Homocoupling polymerization reaction of 2,2',7,7'-tetrabromo-9,9'-spirobifluorene}

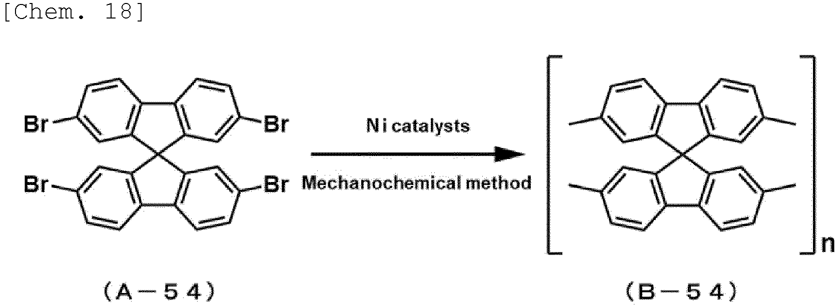

### <Example 52>

To a 5 mL stainless-steel ball mill jar containing stainless-steel balls having a diameter of 10 mm, 63.0 mg (0.2 mmol) of 2,2',7,7'-tetrabromo-9,9'-spirobifluorene (A-54) as a halogen compound, 165.1 mg (0.6 mmol) of bis(1,5-cyclooctadiene)nickel(0) as a nickel catalyst, and 93.7 mg (0.6 mmol) of 2,2'-bipyridine as a heteroatom-containing compound were added under air. The ball mill jar was capped and mounted to a ball mill. While heating was performed at a heat gun set temperature of 160°C (a ball mill jar internal temperature of 80°C), shaking and stirring were performed at 30 Hz for 10 minutes to cause a reaction.

Thereafter, the reaction product was extracted, dried, and purified to obtain 13.1 mg of a homocoupling polymerization reaction product. The homocoupling polymerization reaction product was a polymer having a unit with four bonds represented by (B-54). The isolated yield was 87%.

### {Homocoupling polymerization reaction of 1,3,5-tribromobenzene}

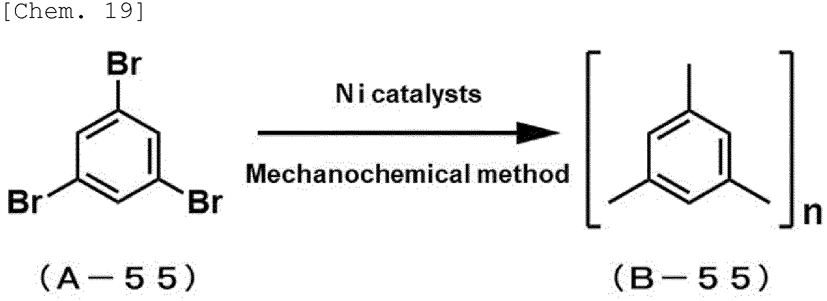

### <Example 53>

To a 5 mL stainless-steel ball mill jar containing stainless-steel balls having a diameter of 10 mm, 31.6 mg (0.05 mmol) of 1,3,5-tribromobenzene (A-55) as a halogen compound, 55.0 mg (0.2 mmol) of bis(1,5-cyclooctadiene)nickel(0) as a nickel catalyst, and 31.3 mg (0.2 mmol) of 2,2'-bipyridine as a heteroatom-containing compound were added under air. The ball mill jar was capped and mounted to a ball mill. While heating was performed at a heat gun set temperature of 160°C (a ball mill jar internal temperature of 80°C), shaking and stirring were performed at 30 Hz for 10 minutes to cause a reaction.

Thereafter, the reaction product was extracted, dried, and purified to obtain 14.4 mg of a homocoupling polymerization reaction product. The homocoupling polymerization reaction product was a polymer having a unit with three bonds represented by (B-55). The isolated yield was 92%.

### {Homocoupling polymerization reaction of 1,2,4,5-tetrabromobenzene}

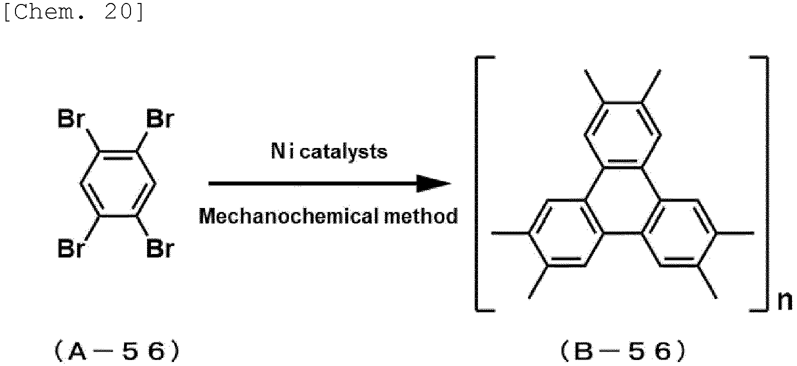

### <Example 54>

To a 5 mL stainless-steel ball mill jar containing stainless-steel balls having a diameter of 10 mm, 78.7 mg (0.2 mmol) of 1,2,4,5-tetrabromobenzene (A-56) as a halogen compound, 220.1 mg (0.8 mmol) of bis(1,5-cyclooctadiene)nickel(0) as a nickel catalyst, and 125.0 mg (0.8 mmol) of 2,2'-bipyridine as a heteroatom-containing compound were added under air. The ball mill jar was capped and mounted to a ball mill. While heating was performed at a heat gun set temperature of 160°C (a ball mill jar internal temperature of 80°C), shaking and stirring were performed at 30 Hz for 10 minutes to cause a reaction.

Thereafter, the reaction product was extracted, dried, and purified to obtain 14.6 mg of a homocoupling polymerization reaction product. The homocoupling polymerization reaction product was a polymer having a unit with six bonds represented by (B-56). The isolated yield was 99%.

### [Regarding Examples]

As demonstrated in Examples 1 to 44, it is understood that the method for producing a homocoupling reaction product according to the present invention is a method that allows a reaction to be carried out under mild reaction conditions by a simple reaction operation without using an organic solvent and can provide a reaction product in a short time in high yield.

As demonstrated in Examples 42 to 44, it is understood that the method for producing a homocoupling reaction product according to the present invention is a method that allows a reaction to be carried out under mild reaction conditions by a simple reaction operation and can provide a reaction product in a short time in high yield also in the case where the halogen species is Br, I, or **Cl.**

As demonstrated in Example 45, it is understood that the method for producing a homocoupling reaction product according to the present invention is a method that allows a reaction to be carried out under mild reaction conditions by a simple reaction operation and can provide a reaction product in a short time in high yield even when the reaction is carried out on a gram scale.

As demonstrated in Example 46, it is understood that among the methods for producing a homocoupling reaction product according to the present invention, the method for producing a homocoupling reaction product using a reductant is a method that can reduce the amount of nickel catalyst used under substantially organic-solvent-free conditions, allows a reaction to be carried out under mild reaction conditions by a simple reaction operation, and can provide a reaction product in a short time in high yield.

As demonstrated in Examples 1 to 45 and 47, it is understood that the method for producing a homocoupling reaction product according to the present invention is a method that allows a reaction to be carried out under mild reaction conditions by a simple reaction operation without using an organic solvent and can provide a reaction product in a short time in high yield, and further allows a wide range of compounds to be used as raw material compounds.

As demonstrated in Examples 48 to 50 and Comparative Examples 1 to 2, it is understood that the method for producing a homocoupling reaction product according to the present invention is a method that allows a reaction to be carried out under mild reaction conditions by a simple reaction operation without using an organic solvent and can provide a high-molecular-weight π-conjugated polymer as a reaction product in a short time in high yield.

As demonstrated in Examples 51 to 54, it is understood that the method for producing a homocoupling reaction product according to the present invention is a method that allows a reaction to be carried out under mild reaction conditions by a simple reaction operation without using an organic solvent and can provide a high-molecular-weight π-conjugated polymer as a reaction product in a short time in high yield, and is further a method that allows a wide range of compounds to be used as raw material compounds and that is usable for various polymerization modes.

Thus, it is understood that the present invention is an invention that is industrially very useful.

## Claims

1. A method for producing a homocoupling reaction product of an organic halogen compound represented by formula (I), the method comprising subjecting the organic halogen compound represented by
formula (I);
A¹-Xₘ (I)
(In formula (I),
A¹ represents an optionally substituted m-valent aromatic hydrocarbon group, an optionally substituted m-valent aromatic heterocyclic group, an optionally substituted **m-**valent heterocyclic group, an optionally substituted **m-**valent aliphatic hydrocarbon group, or an optionally substituted m-valent unsaturated aliphatic hydrocarbon group.
X represents chlorine, bromine, or iodine, and when a plurality of **X's** are present, they may be identical to or
different from each other.
m is a number of X and represents an integer of 1 or more.) to a reaction in presence of a nickel catalyst under a condition (a) and/or a condition (b);
Condition (a): a condition where an amount of a solvent used **is 0.8** mL or less relative to 1 mmol of the organic halogen compound,
Condition (b): a condition where a heteroatom-containing compound is present,
by a mechanochemical method.

2. The method for producing a homocoupling reaction product according to Claim 1, wherein
in formula (I), m is 1 or more and 4 or less, and the homocoupling reaction product is represented by formula (A), formula (P1), formula (P2), or formula (P3);
a homocoupling reaction product represented by formula (A);
A¹-A¹ (A)
(In formula (A), A¹ may be identical or different.),
a linear homocoupling reaction polymerization product, cyclic homocoupling reaction polymerization product, or intramolecular homocoupling reaction product having a repeating unit represented by
formula (P1); 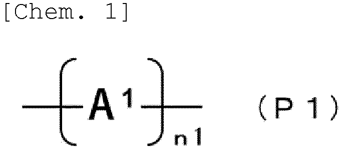
(In formula (P1), a plurality of A¹'s may be identical or different, and n1 is an integer of 2 or more.),
a polymer having a unit represented by
formula (P2); 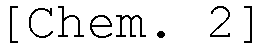 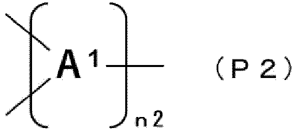
(In formula (P2), a plurality of A¹'s may be identical or different, and n2 is an integer of 2 or more.),
a polymer having a unit represented by
formula (P3); 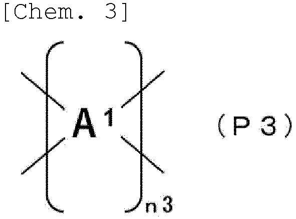
(In formula (P3), a plurality of A¹'s may be identical or different, and n3 is an integer of 2 or more.).

3. The method for producing a homocoupling reaction product according to Claim 1 or 2, wherein the solvent has a melting point of 30°C or lower, and/or the heteroatom-containing compound has a melting point of 50°C or higher.

4. The method for producing a homocoupling reaction product according to Claim 1 or 2, wherein a reductant is further present in the reaction by the mechanochemical method.

5. The method for producing a homocoupling reaction product according to Claim 1 or 2, wherein a temperature in the reaction by the mechanochemical method is 60°C or higher.
